# EUROPÄISCHE PATENTSCHRIFT

(11) **EP 2 612 147 B1**
(45) Veröffentlichungstag und Bekanntmachung des Hinweises auf die Patenterteilung: **30.03.2016**
(21) Anmeldenummer: 11797208.3
(22) Anmeldetag: 11.11.2011
(51) Int. Cl.: G01N 33/531, G01N 33/68, C07H 19/00

(54) **AUTOMATENFÄHIGER IMMUNOASSAY FÜR BIOGENE AMINE**
AUTOMATIC IMMUNOASSAY FOR BIOGENIC AMINES
ESSAI IMMUNOLOGIQUE AUTOMATIQUE POUR AMINE BIOGÈNE

(30) Priorität: 17.11.2010 DE 102010060636
(43) Veröffentlichungstag der Anmeldung: 10.07.2013
(73) Patentinhaber: Kellner, Karl-Heinz, 76131 Karlsruhe (DE)
(72) Erfinder: Kellner, Karl-Heinz, 76131 Karlsruhe (DE)
(74) Vertreter: Benedum, Ulrich Max
(86) Internationale Anmeldenummer: PCT/EP2011/069936
(87) Internationale Veröffentlichungsnummer: WO 2012/065912

(56) Entgegenhaltungen:
- EP-A1- 0 471 345
- EP-A1- 0 668 504
- DE-A1-102005 060 057
- US-A- 4 818 683
- EBERLE A ET AL: "[alpha]-Melanotropin labelled at its tyrosine residue: Synthesis and biological activities of 3'-Iodotyrosine<2>-,3'-<125>Iodotyrosine<2 >- 3',5'-diiodotyrosine<2>-, and (3',5'-<3>H2)tyrosine<2>-[alpha]-melano tropin, and of related peptides", HELVETICA CHIMICA ACTA, VERLAG HELVETICA CHIMICA ACTA, BASEL, CH, Bd. 62, Nr. 7, 1. Januar 1979 (1979-01-01) , Seiten 2460-2483, XP009157638, ISSN: 0018-019X, DOI: 10.1002/HLCA.19790620741

## Beschreibung

### GEBIET DER ERFINDUNG

Die Erfindung betrifft immunologische Verfahren zur Bestimmung niedermolekularer Amine und Aminosäuren in biologischen Substraten.

### HINTERGRUND DER ERFINDUNG

Die Bestimmung körpereigener niedermolekularer Amine und Aminosäuren in Blut, Serum oder Plasma ist für die medizinische Diagnostik von großer Bedeutung, denn einerseits fungieren sie als Mikronährstoffe im Nervensystem und im Gehirn, andererseits beeinflussen sie als erregende und hemmende Neurotransmitter unser Verhalten und unsere Stimmung. Chronische Erkrankungen, Störungen des Magen-Darmtrakts und verschiedene Nahrungsbestandteile können ihre metabolischen Gleichgewichte stören. Eine Bestimmung ist angezeigt in der Diagnostik und Therapie von neuronalen Dysfunktionen wie Schlaflosigkeit, depressiven Störungen, Ess-Störungen (Bulimie, Anorexie), Schizophrenie, posttraumatischem Stresssyndrom, Angstzuständen, Stimmungsschwankungen, Verhaltensstörungen, Schlaganfall, Infarkt, bipolarer Störung, ADHD, Migräne und dergleichen.

Eine fortdauernde Erregung der Nervenzellen durch *Glutamat* führt zu Funktionsverlust und Degeneration. Eine zu geringe Hemmung des Glutamats beeinflusst die Psyche und korreliert mit Stress, Burn-out oder Migräne. L-Glutamat ist auch ein Marker für Größe und Wachstum eines ischämischen Strokes oder Infarkts. *Glutamin* als Vorstufe des Glutamats kann die Blut-Hirn-Schranke passieren. Zu niedrige Glutamin-Spiegel werden gefunden in Patienten mit schweren neurologischen Erkrankungen, Traumata oder katabolischen Zuständen. *gamma-Aminobuttersäure* (GABA) ist ein Antagonist des Glutamats. Sie beeinflusst motorische Kontrolle und Schlaf, und sie ist der wichtigste hemmende Transmitter des zentralen Nervensystems (ZNS). Ein Mangel an GABA geht einher mit chronischem Schmerz, Stress, Schlafstörung, Reizdarm, Schizophrenie, prämenstruellem Syndrom. *Glycin* ist neben Glutamat ein Co-Agonist der NMDA-Rezeptoren und ein Modulator der synaptischen Plastizität. Es besitzt eine wichtige Rolle in den molekularen Vorgängen zu Lernen und Gedächtnis. Ein Glycin-Mangel beeinträchtigt Sprache, Aufmerksamkeit und Lernverhalten, und er wird vorgefunden in Patienten mit Schizophrenie und Zwangsneurosen. *Serotonin* (5-Hydroxytryptamin) ist ein psychoaktiver Neurotransmitter des ZNS. Ein niedriger *Serotonin*-Spiegel beeinträchtigt Laune, Appetit, Schlaf, Lernen und Gedächtnis. Verschiedene neuropsychologische Symptome lassen sich durch Verabreichen der Serotonin-Vorstufe *Tryptophan* mildern. Der *Tryptophan*-Spiegel im Serum dient der Therapiekontrolle und an ihm kann der Serotonin-Spiegel abgelesen werden. Oxidativer Stress führt zur Bildung von Carbonylproteinen (CP) und zum Abbau von *Tryptophan* (Trp) über den Kynureninweg. Das Verhältnis von Carbonylproteinen zu Tryptophan ist ein wichtiger Parameter in der Diagnostik von *Alzheimer*-Erkrankungen*.*

Weitere wichtige Parameter in der Diagnostik sind die Katecholamine, die cholergenen Amine, Oligopeptide wie Vasopressin, Oxytocin oder Angiotensin-I (siehe EP0139552), psychoaktive synthetische Amine wie Methamphetamin und dergleichen, hormonartige Amine wie 5-Methoxytrpytamin (MW = 190), Tyramin (MW = 137), Adrenalin (MW = 183), Octopamin (MW = 153), Noradrenalin, Dopamin, Histamin, Trüodothyronin, Thyroxin, Taurin, symmetrisches (SDMA) und asymmetrischem Dimethylarginin (ADMA) sowie natürliche Aminosäuren wie Phenylalanin, Tyrosin. Patienten mit Nierenversagen besitzen im Plasma niedrige Spiegel von Tyrosin, Threonin, Leucin, Isoleucin, Valin, Lysin oder Histidin, Patienten mit Morbus Crohn, Kolitis oder chronischem Müdigkeitssyndrom anormal niedrige Spiegel von Cystin und Glutamin. Die Menge an asymmetrischem Dimethylarginin (ADMA) im Serum oder Plasma ist ein Prognosewert bei Patienten mit Arteriosklerose, koronarer Herzerkrankung, peripherer arterieller Verschlusskrankheit, Hypertonie, Herzinsuffizienz, Hypercholesterinämie, Nierenerkrankungen oder Diabetes mellitus.

Die Konzentrationen der körpereigenen Amine und Aminosäuren können durch chemische Verfahren bestimmt werden; siehe Methods in Enzymology, XVII, H Tabor and C Tabor Eds. Academic Press, N.Y., 1971; Mefford IN & Barchas JD. (1980) Chromatogr. 181:187-193). Die Konzentration von ADMA wird beispielsweise durch Hochdruck-Flüssigkeitschromatographie (HPLC) bestimmt, da die Isomere ADMA und SDMA zu unterscheiden sind. Die Bestimmung mittels HPLC, Tandem-Massenspektrometrie oder anderer chemischer Verfahren erfordert aber eine aufwändige Probenvorbereitung und Ausrüstung (siehe VanEijk HM et al, Anal Biochem (1999) 271:8-17; Fonath AN et al, Amino Acids (2007) 32:213; US 5,559,038 A; EP2179282 - WO 2009/006338). US 4 818 683 (Morel et al.) beschreibt kompetitive Immunoassays für Histamin, Dopamin und cholergene Amine. Vor ihrer Bestimmung werden die Amine in der Probe quantitativ in höhermolekulare antigene Derivate derivatisiert. Die Derivatisierung erfolgt durch aktivierte Ester wie N-Hydroxysuccinimid oder ein Acylierungsreagenz. Die derivatisierten Aminderivate sind stärker antigen, so dass ihre Konzentration in der Probe über eine Bindungsanalyse an einem Antikörper bestimmt werden kann. Es gibt hunderte Verfahren, Haptene durch Kopplung an einen Träger oder durch Derivatisierung antigen zu machen, siehe Ellman GL, Arch Biochem Biophys (1958) 74:443-450; Riddles PW, Anal Biochem (1979) 94:75-81; Muckerheide A et al., J. Immunol (1987) 138:833; Apple RJ et al, J Immunol (1987) 140:290; Domen PJ, J Immunol (1987) 139:195). Kobayashi N et al, Advances in Clinical Chemistry (2001) 36:139-170. (Abstract) CAPLUS [online], STN Accession 2001: 813015; DE 10 2005 060 057 A1 (Kellner, Immundiagnostik AG); DE 695 20 383T2; EP 0 668 504 A1 (E.I. DuPont); EP 0 471 345 B1 (Boehringer Mannheim Gmbh). EP 0 368 271 B1 (Ishikawa) offenbart derartige Amine direkt durch Umsetzung mit N-Hydroxysuccinimidobiotin zu biotinylieren. Auch ADMA kann durch immunologische Verfahren bestimmt werden (Schulze F et al., Clin Chem Lab Med (2004) 42:1377-1383; Böger RH et al, Clin Chem Lab Med (2003) 41:1467-1472; Böger RH et al., Atherosclerosis (1998) 136:67-77). Weitere reaktive Verbindungen zur Kopplung an Aminen sind offenbart in Eberle A und Hübscher W, Helvetica Chimica Acta (1979) 62(7): 2460-2483.

Die Verfahren für die immunologische Bestimmung von niedermolekularen Aminen (MW < 500 Da) lassen sich aber nicht auf übliche Automaten adaptieren, so dass Reihenuntersuchungen mit großen Probenzahlen nur schwer erfolgen können. Dieses Problem stellt sich besonders bei physiologischen Aminen mit Molmassen kleiner 300 Dalton wie bei ADMA (MW = 202 Da), SDMA (MW 202 Da), L-Glutamat (MW = 147 Da), Glycin (MW= 75) oder GABA (MW = 103). Zudem ist eine höhere Genauigkeit, Zuverlässigkeit und Wirtschaftlichkeit der Nachweisverfahren erwünscht. Der Stand der Technik repräsentiert sich daher als Problem.

### ZUSAMMENFASSUNG DER ERFINDUNG

Offenbart werden automatenfähige Bestimmungsverfahren, Derivatisierungsreagenzien und Derivatisierungsmittel, Kompetitoren und Antikörper zur immunologischen Bestimmung von niedermolekularen Aminen in einer Probe. Bevorzugte Ausführungsformen der Erfindung sind in den Ansprüchen 1 bis 15 beschrieben.

Die Ansprüche betreffen Verfahren zur immunologischen Bestimmung der Konzentration eines niedermolekularen Analyten in einer flüssigen biologischen Probe, wobei der niedermolekulare Analyt eine nicht-peptidische Aminverbindung mit einer Molmasse von weniger als 500 Dalton ist oder eine peptidische Verbindung mit einer reaktiven Aminogruppe und einer Molmasse von weniger als 1500 Dalton, umfassend die Verfahrensschritte: (a) Zusetzen zu einer Probe mit dem niedermolekularen Analyten im stöchiometrischen Überschuss eines Derivatisierungsmittels, das eine chemisch reaktive Gruppe (RG) enthält, die sich quantitativ mit der Aminogruppe (A) des niedermolekularen Analyten umsetzt, wobei das Derivatisierungsmittel in einem Abstand von weniger als zwölf, bevorzugt weniger als acht Kohlenstoffbindungen zur chemisch reaktiven Gruppe (RG) eine Gruppe (GE) mit π-Elektronen oder π-Liganden-Orbitalen aufweist und ausgewählt ist aus Verbindungen der allgemeinen Formeln (la) oder (IIa): 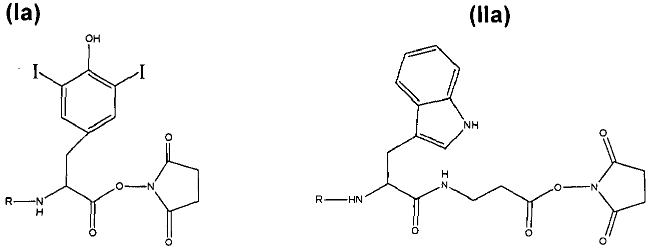 wobei R ein peptidischer Rest ist oder ein Kohlenwasserstoff mit bis zu 40 Kohlenstoffatomen, der Hydroxyl-, Keto- und Ethergruppen enthalten kann sowie wahlfrei N, P, O, oder S.; (b) Umsetzen des Analyten in der Probe der Körperflüssigkeit mit dem Derivatisierungsmittel, optional gefolgt von einem geeigneten Stoppen der Umsetzung mit dem Derivatisierungsmittel, und Erhalt von derivatisierten Aminverbindungen und derivatisiertem Analyt in der Probe der Körperflüssigkeit; (c) Bereitstellen von Antikörpern oder Antikörperfragmenten (AK), hergestellt gegen und spezifisch für den derivatisierten Analyt, wobei die Kontaktstelle zwischen hypervariablen Domänen der variablen Ketten des Antikörpers und dem derivatisierten Analyt die Gruppe (GE) mit den π-Elektronen oder π-Liganden-Orbitalen sowie die Amingruppe (A) nach ihrer Umsetzung mit der reaktiven Gruppe (RG) des Derivatisierungsmittels umfasst; (d) Zusammenbringen der Probe mit dem derivatisierten Analyt mit den spezifischen Antikörpern oder Antikörperfragmenten (AK) und Bestimmen der Menge an derivatisiertem Analyt in der Probe durch Bindungsanalyse, und (e) Bestimmen der Konzentration des niedermolekulaten Analyten in der Probe durch Vergleich mit einer Standardreihe, wobei der Analyt ein biogenes Amin ist und aus der Gruppe mit asymmetrisches Dimethylarginin (ADMA), symmetrisches Dimethylarginin (SDMA), gamma-Aminobuttersäure (GABA), L-Glycin, L-Tryptophan, Amine des Kynureninwegs, L-Arginin, Homocystein, Citrullin, D-Serin, Cystein, Methionin, Carnitin, Phosphoserine, Sulfocystein, Arginobernsteinsäure, Hydroxyprolin, Aspartat, Phosphoethanolamin, Sarcosin (N-Methylglycin), Carnosin (β-Alanyl-L-histidin), Anserine (β-Alanyl-N-methyl-L-histidin), 1,3-Methylhistidin, alpha-Aminoadipinsäure, beta-Alanin, Prolin, Ethanolamin, beta-Aminoisobuttersäure, L-allo-Cystathionin-A, L-Cystathionin, Cystin, allo-Isoleucin, DL-Hydroxylysin, DL-allo-Hydroxylysin, und Homocystin; Serotonin, Histamin; Glutamin; Katecholamine, Dopamin, Metanephrine, Normetanephrine; cholergene Amine, Adrenalin; Oligopeptide und kleinen Peptidhormonen mit bis zu 12 Aminosäuren; Nonapeptide, Vasopressin Oxytocin; Dekapeptide, Angiotensin-I; Oktapeptide, Angiotensin-II; T3 (Trüodothyronin), und T4 (Thyroxin).

Der Analyt ist bevorzugt ausgewählt aus den biogenen Aminen ADMA und SDMA oder den Aminosäuren GABA und Glycin. Histamin ist auch ein biogenes Amin, für das eine genauere und noch raschere Bestimmung gefordert wird.

Das Verfahren kann das Zusetzen im stöchiometrischen Überschuss des Derivatisierungsmittels zu einer Blut-, Serum- oder Plasmaprobe eines Patienten umfassen.

Das verwendete Derivatisierungsmittel enthält prinzipiell in einem Abstand von weniger als zwölf, bevorzugt acht Kohlenstoffbindungen zur chemisch reaktiven Gruppe (RG) eine planare Gruppe (GE) mit konjugierten π-Elektronen. Weiterhin oder alternativ in einem Abstand von weniger als zwölf, vorzugsweise weniger als acht Kohlenstoffbindungen zur chemisch reaktiven Gruppe (RG) ein Halogenatom aus der Gruppe Brom und lod. Das Derivatisierungsmittel enthält somit ein oder mehrere Gruppen, welche gerichtete Wechselwirkungen mit Gruppen der hypervariablen Domänen der variablen Ketten eines Antikörpers eingehen können. Die chemisch reaktive Gruppe (RG) des Derivatisierungsmittels kann ausgewählt sein aus esteraktivierenden und acylierenden Gruppen, N-Hydroxysuccinimid (NHS), Sulfo-NHS, Hydroxybenzotriazol (HOBT), 1-Hydroxy-7-azabenzotriazol (HOAT), Pentafluorphenol, Imidazoliden, Pyridazoliden, aktivierten Arylestern, p-Nitrophenylester, und Aminoalkylcarbonsäuren, worin die Alkylgruppe 2 bis 24 Kohlenstoffatome hat, bevorzugt 2 bis 12 Kohlenstoffatome.

Entsprechend wird als Derivatisierungsreagenz eingesetzt das vorgenannte Derivatisierungsmittel gelöst in einem protischen Lösungsmittel wie DMF oder DMSO. Besonders bevorzugt sind Derivatisierungsreagenzien, die gelöst in DMF oder DMSO Verbindungen der nachstehenden Formel (I) oder (II) enthalten: 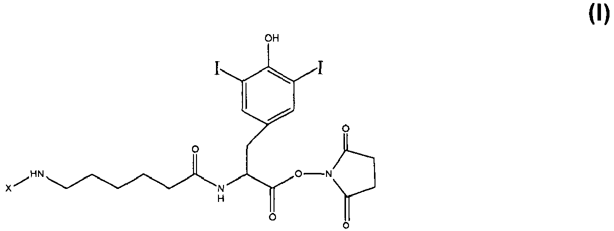 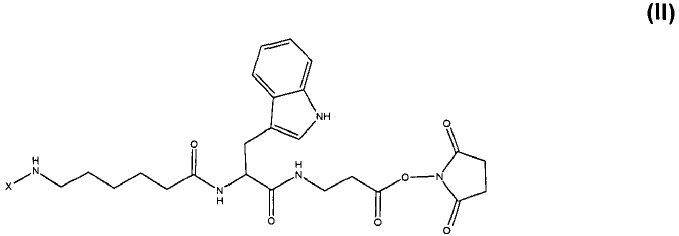 worin X gleich tert-Butyloxycarbonyl (Boc) ist, 9-Fluorenylmethoxycarbonyl (Fmoc), Benzyloxycarbonyl (Cbz), Acetyl, Acyl oder eine Aminschutzgruppe. Ganz besonders bevorzugt sind als Derivatisierungsreagenzien protische Lösungen mit Verbindungen der nachstehenden Formeln (III) und (IV):

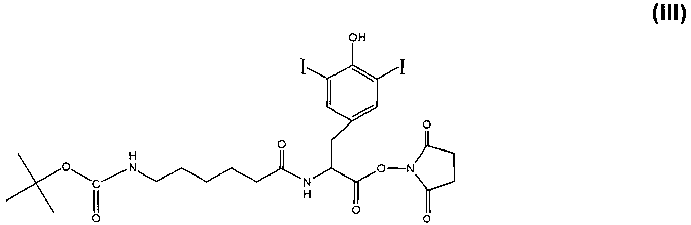

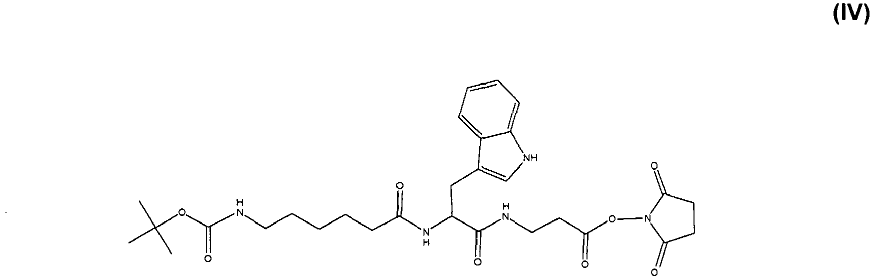

Ein Aspekt betrifft einen Regenziensatz, umfassend das Derivatisierungsreagenz oder eine andere Lösung des Derivatisierungsmittels; einen Kompetitor, erhalten durch Umsetzen des jeweiligen Analyten mit dem Derivatisierungsmittel und gekoppelt an eine Nachweisgruppe oder wahlfrei an eine feste Phase; sowie einen Antikörper, der spezifisch, hochaffin ein Antigen bindet, das erhalten wird bei der Umsetzung des Analyten mit dem Derivatisierungsmittel. Zudem werden offenbart Kompetitor-Verbindungen, welche erhältlich sind durch Umsetzen des Aminanalyten mit einem der vorgenannten Derivatisierungsmittel.

Ein wichtiger Aspekt der Offenbarung sind Antikörper, die an antigene Determinanten binden, welche erhalten werden können durch Umsetzen einer der vorstehend offenbarten Aminanalyten und den jeweiligen Derivatisierungsmitteln. Besonders bevorzugt sind als Bindemoleküle polyklonale Antikörper. Der Antikörper kann auch ein Antikörperfragment beziehungsweise ein scFv-Antikörperfragment. Der Antikörper kann eine Markierung oder eine Nachweisgruppe tragen oder optional auf einer festen Phase immobilisiert sein, bespielsweise, wenn das Nachweisverfahren ein heterogener Assay ist.

Die Derivatisierungsreagenzien eignen sich besonders für Verfahren zur immunologische Bestimmung von ADMA, SDMA, GABA, L-Glutamat, Glycin; sie sind prinzipiell auch geeignet für andere Aminhaptene wie Histamin (MW = 111), L-Tyrosin (MW = 181), L-Phenylalanin (MW = 165), Serotonin (MW = 176), Noradrenalin (MW = 169) oder L-Tryptophan (MW = 204).

Vorteilhaft an den Derivatisierungsmitteln ist, dass die damit erhaltbaren Zielantigene in einem kompetitven Immunoassay, also in Gegenwart eines geeigneten Tracers, für die Herstellung eines Bindungsgleichgewichts an einem gegen das Zielantigen hergestellten Antikörper nicht mehr als zwei Stunden benötigen, selbst bei einer Probentemperatur von Raumtemperatur (18 bis 26°C) bis etwa 37°C, und dies bei 10fach besserer Sensitivität und Spezifität als in den bisherigen Verfahren. Eine so rasche Bindungsanalyse ist mit den bisherigen Derivatisierungsmitteln bei kleinen biogenen Aminen und Aminosäuren nicht möglich. Mit den herkömmlichen Derivatisierungsmitteln für kleine Aminhaptene sind hingegen selbst bei Kühlung der Probe auf 2 bis 8 Grad Celsius Bindungszeiten am Antikörper im Bereich von 12 Stunden bis über Nacht (18 bis 24 Stunden) erforderlich. Diese Chemie eignet sich somit nicht für Automaten und die langen Reaktionszeiten sind problematisch, weil die Reaktionsgefäße (Multititerplatten oder Röhrchen) für so lange Bindungszeiten aus dem Automaten genommen werden. Mit anderen Worten: lange Bindungszeiten führen Fehler ein und sie verlangen einen großen Aufbewahrungsplatz, der in Automaten üblich nicht zur Verfügung steht. Eine Ausnahme ist Histamin dar. Es gibt immunologische Verfahren zur Bestimmung von Histamin, welche Bindungszeiten von nur ca. 3 Stunden verlangen. Aber auch dies ist eine extreme Bindungszeit. Hier geht es um eine weitere Verbesserung der Bindungszeit durch Derivatisierung..

Zu den offenbarten immunologischen Bestimmungsverfahren gehören die Reagenzien für die Derivatisierung der biogenen Amine in der Probe, die Kompetitoren des Zielantigens sowie die Antikörper gegen das Zielantigen. Das Zielantigen umfasst die Amingruppe des biogenen Amins oder der Aminosäure und dazu räumlich benachbart eine eingeführte Derivatgruppe mit π-Elektronen oder eines π-Liganden für ein Pi-Addukt, so dass der Antikörper nicht nur spezifisch, sondern nach Reifung auch hochaffin binden kann. Dadurch wird die Bindungsanalyse schneller.

Die quantitative Derivatisierung in der Probe erfolgt entsprechend mit einem Reagenz, das *nächst* einer chemisch reaktiven Gruppe, die mit dem Aminrest des Analyten reagiert, eine Gruppe mit π-Elektronen oder eines π-Liganden aufweist, so dass nach der Derivatisierung die resultierende Amingruppe und die Gruppe mit den π-Elektronen beziehungsweise des π-Liganden zusammen gemeinsam eine antigene Determinante beziehungsweise ein Hapten bilden, das vom Antikörper spezifisch und hochaffin gebunden werden kann. Die Spezifität eines Antikörpers ergibt sich daraus, wie passgenau die Komplementärregion des Antikörpers zum Zielantigen ist. Die Affinität daraus, wie hoch die Energiebeiträge zur Antikörperbindung sind. Diese Energiebeiträge werden durch die offenbarten Derivatisierungsmittel prinzipiell verbessert, und dies insbesondere bei Aminhapten wie SDMA, ADMA, Glycin oder GABA, wo man selbst auch durch wiederholte Immunisierungen bislang keine hochaffinen Antikörper erhalten kann. Die Energiebeiträge werden durch die offenbarte Derivatisierung erhöht, weil das Zielantigen neben den für die Spezifität notwendigen Aminrest im Bereich der Antikörperbindungsstelle auch π-Elektronenpaare beziehungsweise π-Orbitale eines Liganden enthält. Neben den Energiebeiträgen durch die Van-der-Waals-Kräfte sind hierdurch auch Energiebeiträge durch Pi-Addukte möglich, also durch gerichtete Wechselwirkungen zwischen den π-Orbitalen der neuen antigenen Determinante und den π- und σ-Orbitalen des Antikörpers. Derartige gerichtete Wechselwirkungen treten auch auf zwischen Halogenatomen wie Brom oder lod mit einer Doppelbindung, einer allylischen Gruppe oder einer aromatischen Gruppe. Bei Aminhaptenen wie Histamin, das bereits π-Elektronen enthält, werden durch die erfindungsgemäße Derivatisierung weitere π-Systeme eingeführt, so dass die Affinität der Antikörperbindung insgesamt erhöht wird. Dadurch kann die Einstellung eines guten Bindungsgleichgewichts nochmals beschleunigt werden.

Das Immunogen für die Gewinnung des Antikörpers, an dem die Bindungsanalyse erfolgt, enthält die gleiche antigene Determinante beziehungsweise das gleiche Hapten aus Amin und Derivatgruppe. Das derivatisierte Aminhapten wird hierzu an einen Träger gekoppelt wie BSA (Rinderserumalbumin) oder KLH (Hemocyanin der Lochschnecke - keyhole limpet hemocyanin) und eine Immunantwort in einem Tier erzeugt. Damit die Gruppe mit den π-Elektronen bzw. π-Orbitalen Teil des neuen Haptens wird, muss sie *nächst* der Amingruppe eingeführt werden. *Nächst* steht für einen Abstand von weniger als 12 bis 10 Kohlenstoffbindungen, bevorzugt für weniger als 8 Kohlenstoffbindungen. Idealerweise beträgt der Abstand zwischen Amingruppe und π-Elektronen oder π-Orbitalen etwa 5 bis 6 Kohlenstoffbindungen. Kohlenstoffbindung steht hier für alle Bindungen des Kohlenstoffs mit Kohlenstoff, Stickstoff, Sauerstoff oder Schwefel. Zu Bedenken ist, dass die Oberfläche des neuen Zielantigens nicht linear, sondern dreidimensional ist und mit der Zahl der Kohlenstoffbindungen nur eine nahe Nachbarschaft im Raum umschrieben wird.

Spezifität und Affinität des Antikörpers gegen das Zielantigen sind entscheidend für die Qualität des immunologischen Bestimmungsverfahrens. Bislang versuchte man vor allem eine hohe Antigenität zu erreichen, indem man das Hapten an einen Träger koppelte und bei der Immunisierung ein starkes Adjuvanz hinzusetzte, zum Beispiel komplettes Freundsches Adjuvanz oder *Bordetella pertussis.* Durch wiederholte Immunisierung versuchte man dann die Immunantwort des Tiers in Richtung hochaffiner Antikörper des IgG-Isotyps zu bewegen. Dann selektierte man die generierten Antikörper auf ihre Spezifität gegen die Zielstruktur. Bei biogenen Aminen und kleinen Aminhaptenen konnte man zwar hochspezifische, aber keine hochaffinen Antikörper erhalten, so dass stets lange warten musste, bis sich in einem kompetitiven Immunassay das nötige Bindungsgleichgewicht am Antikörper einstellte. Auch bei bester Immunantwort des Tieres waren die resultierenden Antikörper nicht besonders affin. Durch die offenbarten Derivatisierungsmittel werden die Zielstrukturen am Aminhapten chemisch so ausgelegt, dass sowohl eine hohe Spezifität als auch eine hohe Affinität der Antikörperbindung möglich werden. Die offenbarten Derivatisierungsmittel führen hierzu Gruppen mit π-Elektronen oder π-Orbitalen in enger Nachbarschaft zum kritischen Aminrest ein, damit das immunologische Bestimmungsverfahren an einem Zielantigen erfolgen kann, das wegen seiner chemischen Beschaffenheit höhere Energiebeiträge erlaubt, wenn die Komplementärregion (CDR) eines entsprechend selektierten Antikörpermoleküls daran bindet. Höhere Energiebeiträge sind gegeben, wenn neben den Van-der-Waals-Kräften auch gerichtete Wechselwirkungen mit der Komplementärregion des Antikörpers erfolgen können. Solche gerichteten Wechselwirkungen oder Pi-Addukte sind chemisch auch beobachtbar zwischen Halogenen wie Brom oder lod und aromatischen Systemen, Doppelbindungen oder allylischen Gruppen. Durch mehrfache Immunisierungen mit dem Immunogen (trägergekoppeltem Zielantigen) kann daher die Immunantwort des Tieres zu hochaffinen Antikörpern verlagert werden, so dass die verschiedenen Bindungsbeiträge insgesamt eine hochaffine Bindung ergeben.

Die Spezifität eines Antikörpers ist ein Maß dafür, wie gut ein Antikörper zwischen ähnlichen Strukturen unterscheiden kann. Auf molekularer Ebene wird sie durch die Wechselwirkungen zwischen den variablen Ketten des Antikörpers und dem Analyt bestimmt. Dabei treffen eine Antikörper-Proteinoberfläche und die Analyt-Moleküloberfläche aufeinander, die möglichst genau ineinander passen sollten. Wie nahe sich die zwei Oberflächen dann kommen, bestimmt die Stärke der nichtgerichteten Bindungen. Die Form der Oberfläche und die Anordnung dieser Bindungen entscheiden über die Spezifität. Allerdings sind die Energiebeiträge dieser nicht-gerichteten Bindungen vergleichsweise gering. Kleine biogene Amine wie GABA, Glycin, ADMA oder SDMA lassen deshalb prinzipiell keine hochaffinen Antikörperbindungen zu. Der Energiebeitrag dieser Bindungen kann wie beschrieben erhöht werden, wenn die Komplementärregion des Antikörpers auf eine antigene Determinante trifft, die neben einer Raumstruktur auch gerichtete Bindungen (Pi-Addukte) erlaubt. Dadurch werden im derivatisierten Hapten also erst die Voraussetzungen geschaffen für hohe Energiebeiträge bei der Antikörperbindung. Mit den offenbarten Derivatisierungsmitteln werden zudem Kreuzreaktionen mit anderen Proteinen weitgehend vermieden, da die resultierenden Derivatstrukturen insgesamt ungewöhnlich sind. Kreuzreaktionen werden zudem vermieden durch die resultierende stärkere Bindung und weil die beanspruchten Derivatisierungsreagenzien nicht so groß sind, dass der Unterschied zwischen beispielsweise SDMA und ADMA nicht mehr zum Tragen kommt. Mit einem Derivatisierungsreagenz von mehr als 500 Da geht sehr schnell wieder die Spezifität verloren.

Die Bindungsanalyse erfolgt im beanspruchten Verfahren zwischen einem synthetischen markierten Aminderivat-Kompetitor (Tracer) und dem derivatisierten Amin in der Probe. Wegen der erhöhten Bindungsenergie zwischen Aminderivat und Antikörper kann diese Bindungsanalyse nunmehr bei Raumtemperatur (18 bis 26 Grad Celsius) bis 37 Grad Celsius erfolgen und für die Einstellung des Bindungsgleichgewichts sind regelmäßig nur Bindungszeiten von zwei Stunden oder weniger erforderlich.

Zur Lösung gehört, dass bevorzugt polyklonale Antikörper in der konkurrierende Bindungsanalyse verwendet werden, da dies die Sicherheit der immunologischen Bestimmung erhöht. Polyklonale Antikörper besitzen in der Regel höhere Affinitäten als monoklonale Antikörper. Zudem sind die meisten monoklonalen Antikörper gegen Konformationsepitope gerichtet. Dies schließt aber die Verwendung von monoklonalen Antikörpern oder Antikörper-Fragmenten wie scFv-Fragment nicht aus.

Geeignete Beispiele für Derivatisierungsmittel sind Verbindungen der allgemeinen Formeln (I) oder (II): 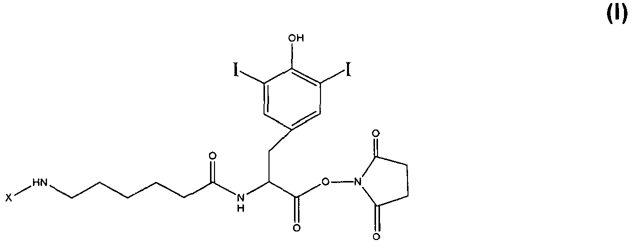 worin ist: X gleich *tert*-Butyloxycarbonyl (Boc), 9-Fluorenylmethoxycarbonyl (Fmoc), Benzyloxycarbonyl (Cbz) oder Boc-Ahx-3,5-Diiodotyrosin-N-hydroxysuccinimid, Acetyl, Acyl oder eine geeignete andere Aminschutzgruppe; oder 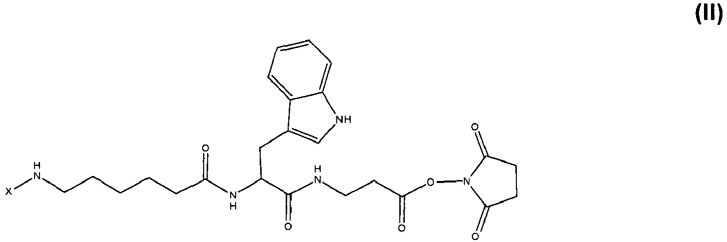 worin ist: X gleich tert-Butyloxycarbonyl (Boc), 9-Fluorenylmethoxycarbonyl (Fmoc), Benzyloxycarbonyl (Cbz) oder Boc-Ahx-3,5-Diiodotyrosin-N-hydroxysuccinimid, Acetyl, Acyl oder eine geeignete andere Aminschutzgruppe.

Anstelle der Schutzgruppe X kann eine Abstandsgruppe mit einer Detektionsgruppe wie Biotin oder Digoxigenin im Derivatisierungsmittel vorliegen. Anstelle von X kann D-Biotinoyl-ε-aminocapronsäure-N-hydroxysuccinimidester (Biotin-7-NHS) im Derivatisierungsmittel eingeführt sein, so dass man den zugehörigen Tracer erhält. Es können auch mehrere N-Aminohexan-Spacer vorhanden sein.

Anstelle der N-Hydroxysuccinimid-Gruppe sind für die Derivatisierung des Amins auch andere chemisch reaktive Gruppe prinzipiell geeignet wie Sulfo-NHS, Hydroxybenzotriazol (HOBT), 1-Hydroxy-7-azabenzotriazol (HOAT), Pentafluorphenol, und dergleichen. Als esteraktivierende Gruppen sind bevorzugt N-Hydroxyestergruppen wie Hydroxysuccinimid, Imidazolide, Pyridazolide, Aminoalkylcarbonsäuren, wobei die Alkylgruppe 2 bis 24 Kohlenstoffatome besitzen kann, oder aktivierte Arylestergruppen wie p-Nitrophenylester.

Ein bevorzugtes Derivatisierungsmittel ist Boc-Ahx-3,5-Tyr-NHS (Boc = *tert.-*Butyloxycarbonyl; Ahx = Aminohexansäure; 3,5-Tyr = 3,5-Diiodotyrosin; NHS = N-Hydroxysuccinimid) gemäß nachstehender Formel (III):

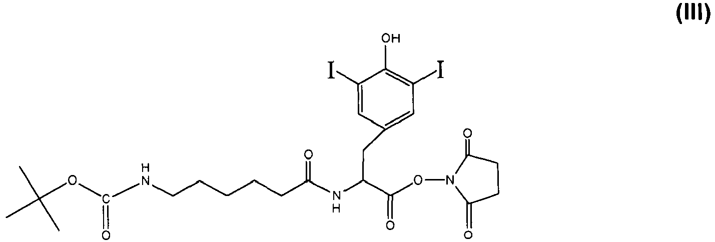

Ein weiteres bevorzugtes Derivatisierungsmittel ist Boc-Ahx-Trp-β-Ala-NHS (Boc = tert. Butyloxycarbonyl; Ahx = Aminohexansäure; β-Ala = beta-Alanin; NHS = N-Hydroxysuccinimid) gemäß nachstehender Formel (IV):

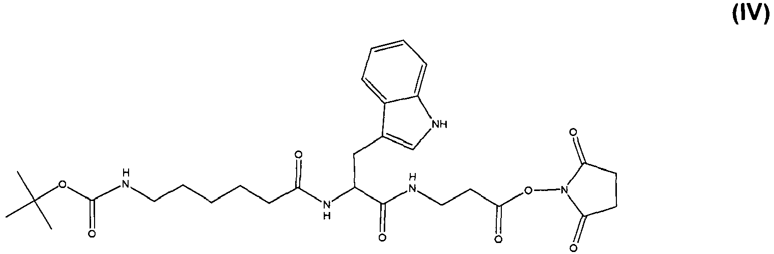

Die vorgestellten Derivatisierungsmittel enthalten nächst der als beispielhaft genannten chemisch reaktiven Gruppe (NHS) mit 3,5-Diiodotyrosin und Trp-β-Ala antigene Determinanten, die körperfremd sind in Bezug auf Mammalia, die sterisch anspruchsvoll sind und die wegen der π-Elektronen in einer festen Raumstruktur zum derivatisierten Aminrest stehen werden, so dass in der konkurrierenden Bindungsanalyse das Zielantigen wegen der Möglichkeit gerichteter Wechselwirkungen einen hohen Energiebetrag zur Affinität des Antikörpers beitragen kann. Der Abstand der beiden kritischen Strukturgruppen - π-Elektronen und derivatisierte Amingruppe - sollte im Zielantigen nicht über mehr als zwölf Bindungen gehen, da sonst beide Gruppen nicht innerhalb der einen Kontaktstelle des Antikörpers liegen.

Im Derivatisierungsmittel von Formel III ist die antigene Gruppe mit den π-Elektronen das planare 3,5-Diiodotyrosin. Im Derivatisierungsmittel von Formel IV ist die antigene Gruppe mit den konjugierten π-Elektronen das Dipeptid aus Tryptophan und beta-Alanin. Beta-Alanin kommt in Mammalia nicht vor, sondern nur in Pathogenen (Viren und Bakterien), so dass das Dipeptid stark immunogen ist.

Es sind weitere Varianten der antigenen Determinanten am Derivatisierungsmittel denkbar. So kann die Tyrosin- und die Tryptophan-Gruppen beliebig substituiert sein und andere Substituenten tragen Auch kann die antigene Determinante mit den π-Elektronen ein konjugiertes Ringsystem mit ein, zwei oder mehreren Substituenten sein. Geeignet sollten auch Farbstoffgruppen sein wie Acridin und Fluorescein, wobei hier funktionalisierte Verbindungen zur Verfügung stehen wie Fluorescein-isothio-cynanat(FITC) und die Succinimidylester des Fluoresceins und Acridins. Bei der Kopplung so großer Ringsysteme kann der Nachteil auftreten, dass diese mehrere Epitope für die Bindung von Antikörpern bereitstellen. Gewünscht ist jedoch bei der Derivatisierung die Schaffung eines (1) spezifischen Epitops oder Haptens mit einer chemischen Struktur, welche eine hochaffine Antikörperbindung erlaubt beziehungsweise deren chemische Struktur einen hohen Energiebeitrag zur Antikörperbindung leisten kann. Sehr große Derivatgruppen führen hingegen zu einer geringen Derivatisierungseffizienz, zu Löslichkeitsproblemen und sie begünstigen das Auftreten von Kreuzreaktionen, was wiederum die Qualität des immunologische Bestimmungsverfahrens insgesamt beeinträchtigt.

Wird das Derivatisierungsmittel gemäß Formel III in einem Immunoassay zur Bestimmung von gamma-Aminobuttersäure (GABA) eingesetzt, können die Antikörper gegen das Hapten zum Beispiel durch Koppeln des Haptens an KLH und Immunisieren mit KLH-DSS-3,5-Diiodotyrosin-GABA gemäß Formel V erhalten werden.

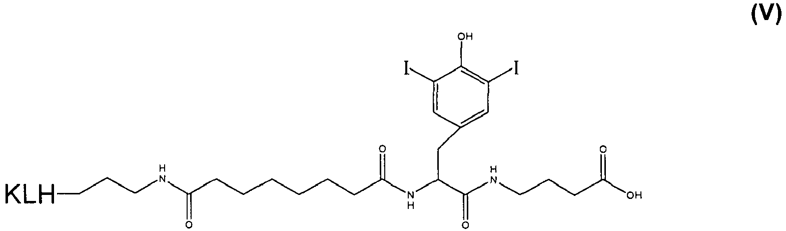

Das Immunogen wird erhalten durch Peptidkopplung von gamma-Aminobuttersäure mit 3,5-Diiodotyrosin und Koppeln des Dipeptids an KLH durch Umsetzen mit Disuccinimidylsuberat (DSS = Disuccinimidyloctandisäure). Die Derivatisierung des Amins in der Probe (Serum oder Plasma) erfolgt in diesem Beispiel mit Boc-Ahx-3,5-Diiodotyrosin-NHS (NHS = N-Hydroxysuccinimid; Ahx = Aminohexansäure), so dass als Analyt und Zielantigen Boc-Ahx-3,5-Tyr-GABA gemäß Formel VI erhalten wird:

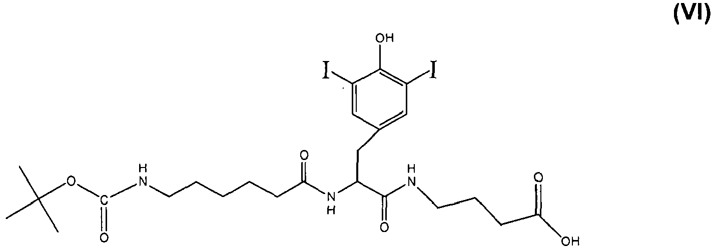

Bei der Immunisierung mit dem Immunogen von Formel V werden Antikörper hoher Affinität und Spezifität gegen die GABA-Derivatstruktur (VI) erhalten, so dass die Bindungsreaktion rasch und bei Raumtemperatur erfolgen kann. Nicht nur der Antikörper liefert dann hohe Spezifität, sondern auch die bei der Derivatisierung hergestellte antigene Determinante besitzt eine chemische Struktur, welche Spezifität und hohe Affinität erlaubt.

Für direkt bestimmende Verfahren kann das Derivatisierungsreagenz anstelle von Boc eine Detektionsgruppe wie Biotin enthalten, zum Beispiel wie in Biotin-Ahx-Ahx-3,5-Diiodotyrosin-NHS oder Biotin-Ahx-Ahx-Trp-β-Ala-NHS. Die Detektionsgruppe ist dann bevorzugt ausgewählt aus Biotin, Digoxigenin, Desthiobiotin, substituierten und derivatisierten Aminosäuren, Oligopeptiden, Fluoreszeinisothiocyanat (FITC), Avidin, Streptavidin, Protein-A, Protein-G und dergleichen. Die Detektionsgruppe kann auch eine fluoreszierende, lumineszierende oder eine farbgebende Gruppe sein, beispielsweise eine Enzymgruppe.

Das offenbarte Verfahren eignet sich für kleine Monoamine, insbesondere für natürliche und nicht-natürliche Aminosäuren, asymmetrisches Dimethylarginin (ADMA), gamma-Aminobuttersäure (GABA), L-Glycin, L-Tryptophan, L-Arginin, Homocystein, Citrullin, D-Serin, Cystein, Methionin, Carnitin, Phosphoserine, Sulfocystein, Arginobernsteinsäure, Hydroxyprolin, Aspartat, Phosphoethanolamin, Sarcosin (N-Methylglycin), Carnosin (β-Alanyl-L-histidin), Anserine (β-Alanyl-N-methyl-L-histidin), 1,3-Methylhistidin, alpha-Aminoadipinsäure, beta-Alanin, Prolin, Ethanolamin, beta-Aminoisobuttersäure, Cystein, L-allo-Cystathionin-A, L-Cystathionin, Cystin, allo-Isoleucin, DL-Hydroxylysin, DL-allo-Hydroxylysin, und Homocystin; biogene Amine wie Serotonin, Histamin; Glutamin; Katecholamine, Dopamin, Metanephrine, Normetanephrine; cholergene Amine wie Adrenalin; Oligopeptide und kleinen Peptidhormonen mit bis zu 12 Aminosäuren; Nonapeptide, Vasopressin Oxytocin; Dekapeptide, Angiotensin-I; Oktapeptide, Angiotensin-II; T3 (Triiodothyronin), und T4 (Thyroxin). Je komplexer aber der Analyt, desto weniger besteht Bedarf es einer Derivatisierung und der Einführung von chemischen Strukturen für die Generierung eines Zielantigens mit einem hohen Beitrag zur Bindung am Bindeprotein (Antikörper).

Die Erfindung umfasst weiterhin Reagenziensätze (Kits) zur Durchführung des Verfahrens, insbesondere einen Chemikaliensatz für die Derivatisierung der Probe, Antikörper beziehungsweise Antikörperfragmente gegen das generierte Zielantigen sowie Reagenzien für die Detektion.

### BESCHREIBUNG DER ABBILDUNGEN

Weitere Vorteile, Merkmale und Ausführungsformen der Erfindung ergeben sich aus den Beispielen und den anliegenden Abbildungen. Es zeigt:
- Fig. 1: ein Diagramm mit einem Vergleich der Sensitivität der Antikörperbindungen für ein herkömmliches ADMA-Zielantigen und ein ADMA-Zielantigen gemäß der Offenbarung;
- Fig. 2: Diagramm mit einer Korrelation der Werte aus dem indirekt kompetitiven ADMA-ELISA gemäß der Offenbarung und Werten durch HPLC-MS;
- Fig. 3: Diagramm mit einer Korrelation der Werte aus dem direkt kompetitiven ADMA-ELISA gemäß der Offenbarung und Werten durch HLPC-MS;
- Fig. 4: Diagramm mit einer Standardkurve für die Bestimmung von Histamin in Serum.

### EINGEHENDE BESCHREIBUNG

"Analyt" im Sinne vorliegenden sind kleine physiologisch aktive Amine, die vor ihrer Derivatisierung im Wesentichen keine antigenen Eigenschaften besitzen.

Die Proben sind Körperflüssigkeiten und Blutproben, insbesondere flüssige Blutfraktionen wie Serum oder Plasma. Die Probe kann eine biologische Flüssigkeit sein wie Speichel oder Urin oder ein solubilisierter Gewebsextrakt. Das Verfahren hat besondere Bedeutung für die Bestimmung von physiologisch aktiven Aminen in biologischen Flüssigkeiten, deren Bestimmung mit sonstigen immunologischen Verfahren problematisch ist. Es ist nicht auf die Bestimmung biogener Amine beschränkt, sondern es umfasst auch die Bestimmung von Arzneimitteln, Drogen oder anderer synthetischer Substanzen in beliebigen protischen Systemen. Typische Vertreter physiologisch aktiver Amine, welche selbst nicht antigen sind, sind Aminosäuren wie asymmetrisches Dimethylarginin (ADMA), symmetrisches Dimethylarginin (SDMA), gamma-Aminobuttersäure (GABA), L-Glycin, L-Arginin, Homocystein, Citrullin, D-Serin, Cystein, Methionin, Tryptophan, Carnitin, Phosphoserine, Sulfocystein, Arginobernsteinsäure, Hydroxyprolin, Aspartat, Phosphoethanolamin, Sarcosin (N-Methylglycin), Carnitin, Carnosin (β-Alanyl-L-histidin), Anserine (β-Alanyl-N-methyl-L-histidin), 1,3-Methylhistidin, alpha-Aminoadipinsäure, beta-Alanin, Prolin, Ethanolamin, beta-Aminoisobuttersäure, Cystein, L-allo-Cystathionin-A, L-Cystathionin, Cystin, Homocystein, Citrullin, allo-Isoleucin, DL-Hydroxylysin, DL-allo-Hydroxylysin, und Homocystin; Serotonin, Histamin; Glutamin; Katecholamine wie Dopamin, Metanephrine, Normetanephrine; cholergene Amine wie Adrenalin. Wenn nachstehend das Verfahren anhand einzelner Amine erläutert wird, so dient dies der Darstellung, wie die Kompetitoren und Derivatisierungsreagenzien einzusetzen sind. Die Offenbarung ist nicht auf die beschriebenen Ausführungsformen beschränkt.

Die grundsätzliche Bekanntheit immunologischer Bestimmungsverfahren wird vorausgesetzt. Sie lassen sich nach Umsetzung und Nachweisverfahren verschiedenen Typen zuordnen. Zu den kompetitiven Bestimmungsvefahren gehören der Radioimmunoassay (RIA) und viele ELISA (enzyme-linked immunosorbent assay), die je nach Markierung, Art des zu bestimmenden Moleküls und der Bestimmung in homogener oder heterogener Phase noch einmal verschiedenen Untertypen zugeordnet werden. Prinzipiell wird bei den kompetitiven Verfahren der zu untersuchenden Probe eine bekannte Menge Kompetitor (Tracer) zugesetzt sowie ein Unterschuss eines spezifischen Binders (Antikörpers), wobei in der Regel eine der Komponenten - Kompetitor oder Binder (Antikörper) - markiert ist und der andere an einer festen Phase gebunden. Man ermittelt dann die Menge des Analyten in der Probe aus der Menge der an die feste Phase gebundenen Markierung und zwar zumeist über eine Eichkurve mit einer Reihe bekannter Konzentrationen des Analyten.

Sandwich-Bestimmungsverfahren werden normalerweise für Analyten eingesetzt, die zwei beabstandete Bindungsstellen für Binder wie z.B. zwei verschiedene Antikörper aufweisen.

Auch nach Derivatisierung können im Stand der Technik die Antikörper gegen die derivatisierten Aminhaptene in der Regel mit nur geringer Affinität binden, so dass sich die Bindungsgleichgewichte erst nach langer Zeit, 12 bis 18 Stunden, bei niederen Probentemperaturen einstellen. Die herkömmlichen Bestimmungsverfahren sind somit nicht auf Automaten adaptierbar. Stand- und Reaktionszeiten von mehr als zwei Stunden sind in Automaten schon wegen des Platzbedarfs bei hohen Probenzahlen nicht tragbar. Auch ist eine höhere Sensitivität und Spezifität des Nachweisverfahrens verlangt. In dem offenbarten Verfahren werden die physiologisch aktiven Amine so derivatisiert, dass nächst des Aminrests Gruppen mit π-Elektronen eingeführt werden, so dass die derivatisierte Aminstruktur zusammen mit den π-Elektronen ein Hapten beziehungsweise eine antigene Determinante bildet, welche eine hochaffine spezifische Antikörperbindung erlaubt. Das Verfahren ist bevorzugt ein umgekehrt kompetitiver Bindungsassay. Es wird hierbei in der Probe faktisch nicht die Menge an Zielantigen (Aminderivat), sondern die Menge an Kompetitor (Tracer) bestimmt, der spezifisch am Antikörper gebunden ist und nicht vom derivatisierten Zielantigen aus der Bindung am Antikörper verdrängt wurde. Alternativ werden bei der "direkten" Bestimmung bei der Derivatisierung neben der antigenen Gruppe mit π-Elektronen noch beabstandet dazu eine Gruppe für den Nachweis des Derivats eingeführt. Dieses Vorgehen ist weniger bevorzugt, da mit jeder weiteren Gruppe die Probleme mit der Derivatisierungseffizienz oder der Löslichkeit der resultierenden Derivate sprunghaft steigen.

Als aktivierende Gruppen im Derivatisierungsreagens sind geeignet N-Hydroxyestergruppen wie N-Hydroxysuccinimid, Imidazolide, Pyridazolide, Aminoalkylcarbonsäuren, wobei die Alkylgruppe 2 bis 24 Kohlenstoffatome besitzen kann, oder aktivierte Arylestergruppen wie p-Nitrophenylester. Ketogruppenhaltige Analyten werden bevorzugt mit einem Hydrazin-Derivatisierungsreagens umgesetzt. SH-haltige Analyten werden bei Bedarf mit einem Maleinimido-Derivatisierungsreagens umgesetzt. Die Analyten in der Probe werden in organischen Medien wie DMSO oder Dimethylformamid (DMF) unter Zusatz von Triethylamin oder in Puffergemischen, bevorzugt Kaliumphosphatpuffer, pH 7,5 oder höher, mit einem Überschuss Derivatisierungsreagenz umgesetzt.

Da die Konzentration an Analyt in der Probe sehr niedrig ist und das Zielantigen durch Umsetzung mit dem Derivatisierungsreagenz gebildet werden muss, wird in der Regel die am Antikörper gebundene Menge an Kompetitor (Tracer) bestimmt. In der Hauptausführungsform betrifft das beanspruchte Verfahren einen invers kompetitiven Immunoassay mit vorheriger Derivatisierung des Analyten zum Zielantigen. Die Markierung für den Nachweis des Tracers am Antikörper kann beispielsweise ein Enzym sein wie Peroxidase, ein Fluoreszenz- oder Chemilumineszenz-Marker, ein Elektrochemilumineszenz-Marker beziehungsweise ein Ruthenium-Komplex oder unter Umständen auch eine radioaktive Markierung.

### BEISPIEL

### Beispiel 1 - Immunologische Bestimmung von GABA in Blutplasma

### a) Derivatisierung der Probe

Die immunologische Bestimmung von gamma-Aminobuttersäure (GABA) in Blutplasma erfolgte mit Boc-Ahx-3,5-Tyr-GABA (Boc = *tert.* Butyloxycarbonyl; Ahx = Aminohexansäure; 3,5-Tyr = 3,5-Diiodotyrosin) gemäß Formel VI als Antigen. Die Derivatisierung der Amine in der Plasmaprobe erfolgte bei Raumtemperatur im stöchiometrischen Überschuss mit dem Derivatisierungsreagenz Boc-Ahx-3,5-Tyr-NHS gemäß Formel III, gelöst in Dimethylsulfoxid (DMSO). Im Einzelnen wurde 50 µL Probe (Serum, Standard beziehungsweise Kontrolle) mit 150 µL Phosphatpuffer (pH 8,0) zusammen pipettiert und 50 µL 50 mM Boc-Ahx-3,5-DÜodotyrosin-NHS (III), gelöst in DMSO zugesetzt, so dass im Reaktionsansatz ein etwa 5facher Überschuss an Derivatisierungsreagenz vorlag, bezogen auf die im Serum vorhandenen primären und sekundären Aminogruppen (ca. 2 mM Aminosäuren, <1 mM Amine, 5-10 mM Amin im Humanserumalbumin). Die Derivatisierung wurde nach 30 Minuten durch Zusatz von 250 µl 1%iger BSA gestoppt. Nach weiteren 30 Minuten bei Raumtemperatur (18 bis 26 Grad Celsius) wurde 50 µL derivatisierte Probe (1:10 verdünnt) in die immunologische Bestimmung eingesetzt.

### b) Antikörper gegen Zielanalyt gemäß Formel V

Spezifische polyklonale Antikörper gegen das Zielantigen wurden gewonnen durch Kopplung des Haptens 3,5-Diiodotyrosin-GABA mit Hilfe von Disuccinimidylsuberat (DSS = Disuccinimidyloctandioat) an KLH und Immunisierung mit KLH-DSS-3,5-Diiodotyrosin-GABA gemäß Formel V.

Mit dieser Verbindung als Immunogen erhielt man in Kaninchen nach Immunisierung mit Freundschem Adjuvanz und Boostern Anti-KLH-Antikörper, anti-KLH-DSS-3,5-Diodotyrosin-Antikörper und hochaffine spezifische Antikörper gegen 3,5-Diiodotyrosin-GABA. Im Zielantigen kommen somit die für eine hohe Affinität vorteilhafte chemische Beschaffenheit des Diiodotyrosin-Rests mit der Analytengruppe GABA in einer antigenen Determinante zusammen. Diese Wechselwirkungen sind vom pH, der Temperatur und der Salzkonzentration des umgebenden Mediums abhängig. Das Immunogen (Formel V) und das derivatisierte Zielantigen (VI) sind nur in der relevanten Analysestruktur gleich, ansonsten verschieden. Die nach Kopplung an einen Träger erzeugten polyklonalen Kaninchen-Anti-3,5-Tyr-GABA-Antikörper wurden in üblicher Weise über eine IgG-Säule isoliert und affinitätsgereinigt.

### c) Kompetitiver Immunassay zur Bestimmung von GABA in Blutplasma

Für den ELISA wurde eine Multititerplatte (MTP) mit Streptavidin beschichtet. Als Tracer im kompetitiven Bindungsassay diente Biotin-Ahx-Ahx-3,5-Diiodotyrosin-GABA gemäß Formel VII 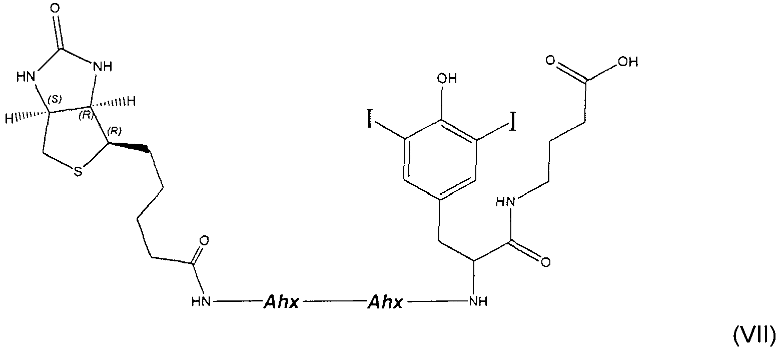 erhalten durch Umsetzen von 3,5-Diiodotyrosin-GABA mit Biotin-Ahx-Ahx-NHS in DMSO. Die Mikrotiterplatte bindet somit den Systemantikörper über MTP/Streptavidin/-Biotin-Ahx-Ahx-3,5-Diiodotyrosin-GABA-AK in Abhängigkeit von der Konzentration an Boc-Ahx- 3,5-Diiodotyrosin-GABA-Derivat (Formel VI) in der Probe.

Im Einzelnen wurde für die kompetitive Bestimmung 50 µl derivatisierte Probe (VI) aus (a) zusammen mit einer definierten Menge Tracer Biotin-Ahx-Ahx-3,5-Diiodotyrosin-GABA (VII) und 50 µl verdünnter (im Unterschuss) polyklonaler Kaninchen-Anti-3,5-Diodotyrosin-GABA-Antikörper aus (b) zwei Stunden bei Raumtemperatur in der Streptavidin-beschichteten MTP inkubiert, die MTP mit Phosphatpuffer (pH = 8,0) mehrfach gewaschen, dann eine Stunde bei Raumtemperatur (18-26°C) HRPkonjugierter Sekundärantikörper zugesetzt, erneut gewaschen und konventionell mit Tetramethylbenzidin (TMB) entwickelt.

Die Vorteile gegenüber herkömmlichen ELISAs lagen darin, dass (i) keine Extraktion von GABA erforderlich war, (ii) das nötige Probevolumen nur 50 µl betrug und dies bei einer Nachweisgrenze von 25 nmol/L. Dies ist für GABA als Molekül mit weniger als 100 Da sehr gut, zumal GABA sterisch selbst nicht anspruchsvoll ist. Durch die Wahl der Aminosäuren sowie der Boc-Schutzgruppe ist der NHS-Ester (III) gut in DMSO löslich und gleichzeitig gut löslich in der wässrigen Probe, so dass das Verfahren voll automatenfähig war, zumal alle Reaktionen schon in der Versuchsphase weniger als zwei Stunden benötigten.

### Beispiel 2 - Indirekte immunologische Bestimmung von ADMA

### a) Testprinzip

Der ADMA-Test basiert auf der Methode des indirekt kompetitiven Enzymimmunoassay. Die Probe mit der unbekannten ADMA-Konzentration wird mit Reaktionspuffer und Derivatisierungsreagenz versetzt und zum ADMA-Zielantigen derivatisiert. In dem Reaktionspuffer ist eine bekannte Menge markiertes ADMA-Derivat als Tracer enthalten sowie ein Unterschuss polyklonaler Systemantikörper gegen das ADMA-Zielantigen (ADMA-Derivat). Die derivatisierte Probe wird dann mit einer ELISA-Platte als stationäre Phase inkubiert, die mit Sekundärantikörper gegen die System-Immunglobulin beschichtet ist. Während der Inkubation konkurriert das ADMA-Zielantigen in der Probe mit dem Tracer um die Bindung an dem polyklonalen Systemantikörper, der dann vom Sekundärantikörper auf der festen Phase gebunden wird. Hierbei verdrängt das Zielantigen in der Probe den Tracer aus der Bindung an dem Systemantikörper. Somit ist die Konzentration des an den Antikörper gebundenen Tracers umgekehrt proportional zur Konzentration des Zielantigens in der Probe. Nach dem Abwaschen ungebundener Komponenten von der festen Phase wird die Menge an Tracer auf der Platte durch eine chromogene Reaktion nachgewiesen.

### b) Reagenzien der Testpackung

1. Lösung mit polyklonalen Kaninchen-Antikörpern gegen KLH-DSS-Trp-β-Ala-ADMA;
2. Lösung mit 50 mM Boc-Ahx-Trp-β-Ala-NHS in DMSO (Derivatisierungsreagenz);
3. 1%ige BSA in 10 mM Kaliumphosphatpuffer (pH 8,0) als Stopplösung:
4. Wasch- und Assaypuffer (50mM/L NaH₂PO₄/Na₂HPO₄-Puffer, pH 7,5, 2% Gelatine, 5 mM/L EGTA, 2 mM β-Mercaptoethanol)
4. Reaktionpuffer mit Biotin-Ahx-Ahx-Trp-β-Ala-ADMA als Kompetitor (Tracer)
5. Mikrotiterplatte als feste Phase, beschichtet mit Anti-Kaninchen-IgG-Antikörper
6. Lösung mit Meerrettichperoxidase-konjugiertem Streptavidin
7. Entwicklungsreagenz (Tetramethylbenzidin)

Die polyklonalen Kaninchen-Antikörper wurden gewonnen durch Immunisieren gegen das Immunogen: KLH-DSS-Trp-β-Ala-ADMA. Nach mehrfachen Boostern der Immunantwort wurden die mit dem Serum gewonnenen Antikörper an einer IgG-Säule affinitätsgereinigt.

### c) Derivatisierung

Für die Vorbereitung wurde (i) 50 µl Probe mit 150 µl 10 mM/L Kaliumphosphatpuffer (pH 8,0) verdünnt, dann (ii) 50 µl 50mM/L Boc-Ahx-Trp-β-Ala-NHS in DMSO zugegeben, gemischt und bei Raumtemperatur inkubiert. Nach 30 Minuten wurde die Derivatisierung durch Zusatz von 1%iger BSA-Lösung gestoppt. Die derivatisierte Probe wurde dann direkt in einem heterogenen kompetitiven Immuno-assay eingesetzt.

### d) Kompetitiver ELISA

50 µl derivatisierte Probe, 50 µl Biotin-Ahx-Ahx-Trp-β-Ala-ADMA (Tracer) und 100 µl polyklonaler Anti-Trp-β-Ala-ADMA-Antikörper (im Unterschuss) wurden in Assaypuffer bei Raumtemperatur (18 - 26°C) in einer mit Sekundärantikörper beschichteten Mikrotiterplatte eine Stunde inkubiert. Die feste Phase wurde mit Waschpuffer gewaschen und die Menge des an der festen Phase gebundenen Tracers bestimmt durch Inkubation (0,5 Std) mit 100 µL HRP-konjugiertem Streptavidin (1:50000 in Waschpuffer, entsprechend ca. 100 ng) und Entwickeln mit 100 µl Tetramethylbenzidin(TMB)-Substrat-Lösung (NOVUM Diagnostika GmbH, DE) bei Raumtemperatur (18-26°C) im Dunkeln. Nach 15 Minuten wurde die Farbentwicklung durch Zugabe von 50 µl 2 M H₂SO₄ gestoppt. Die Messung der optischen Dichte erfolgte sofort im Anschluss, wobei die Extinktion im Mikrotiterplatten-Photometer (ELISA-Reader) bei 450 nm gegen die Referenzwellenlänge 620 nm (oder 690 nm) gemessen wurde. Die Gesamtdauer der Bestimmung betrug wegen der hohen Affinität und Sensitivität der Antikörper gegen das Zielantigen nur noch etwa 2 Stunden und kann damit auf Automaten adaptiert werden; siehe nachstehende Tabelle I:

**TABELLE I**

| *Vergleich der Bindung an ADMA-Zielantigenen* | | | |
|---|---|---|---|
| ADMA-Trp-βAla-Antiqen | | ADMA-Acetyl-Antigen | |
| Konz. in µMol/L | OD | Konz. In µMol/L | OD |
| 0,00001 | 1 | 0,0001 | 1 |
| 0,0014 | 0,85 | 0,01 | 0,9 |
| 0,0035 | 0,63 | 0,025 | 0,75 |
| 0,0071 | 0,47 | 0,05 | 0,57 |
| 0,0142 | 0,35 | 0,1 | 0,43 |
| 0,0285 | 0,22 | 0,2 | 0,24 |
| Sensitivitätsverbesserung Faktor 12,5 | | | |

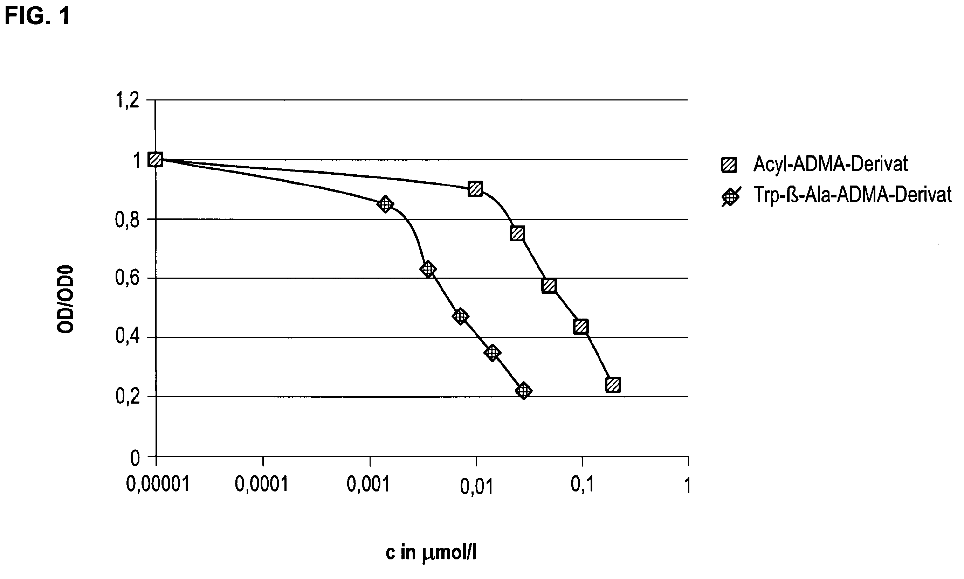

Die Vorteile gegenüber bekannten kommerziellen Immunoassays mit einem N-Acyl-ADMA als Zielantigen (DLD Diagnostika GmbH, Hamburg, DE) waren, wie Tabelle I und graphische Auswertung (Figur 1) zeigen, eine 12 bis 15-fach höhere Sensitivität bei Raumtemperatur und 1 Std. Bindungszeit.

### e) Ergebnisse mit Normalproben

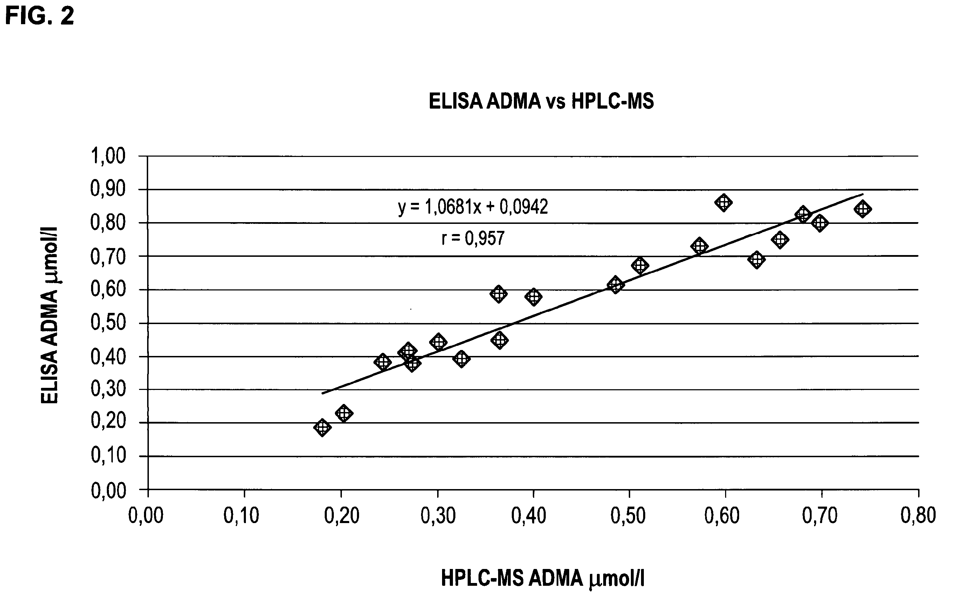

Figur 2 zeigt eine Korrelation der immunologisch bestimmten Werte gegenüber Werten durch HPLC-MS als Referenz. Die Korrelation mit den HPLC-MS-Werten betrug r=0,95 (n=19). Die physiologische Konzentration von ADMA im Serum beträgt normalerweise etwa 0,3 bis 0,5 µMol/L.

### Beispiel 3 - Direkte kompetitive Bestimmung von ADMA

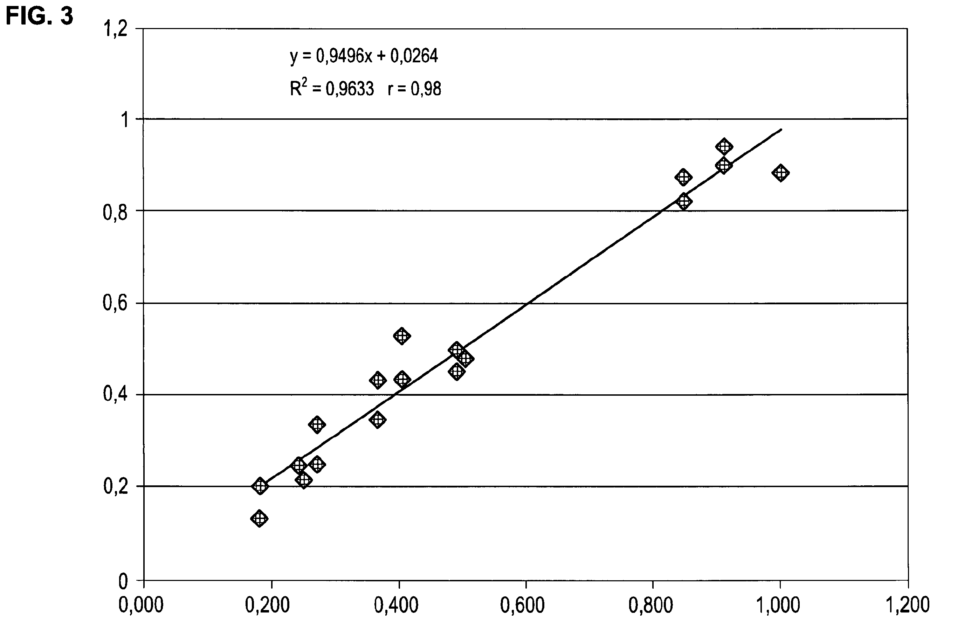

Der Versuch aus Beispiel 2 wurde wiederholt, nur dass der polyklonale Systemantikörper gegen das Zielantigen direkt auf die feste Phase aufgebracht war. Figur 3 zeigt die Korrelation der immunologisch ermittelten Werte mit Werten durch HPLC-MS. Die Korrelation betrug r=0,98 (n=18)

Eine Studie mit Plasmaproben von augenscheinlich Gesunden (n = 70) ergab für den hier beschriebenen Test mit dem erfindungsgemäßen Zielantigen einen Plasma-Mittelwert von ca 0,45 µMol/L bei einer Standardabweichung von 0,09 µMol/L. Der Normalbereich lag zwischen 0,26 und 0,64 µMol/L. Es wird aber jedes Labor seinen eigenen Normalwerte-Bereich ermitteln müssen, weil die Referenzbereiche von der Auswahl des Probandenkollektivs abhängig sind. Die hier angegebenen Normalbereiche dienen der Orientierung. Die ermittelte Nachweisgrenze lag bei 0,04 µMol/L

Die *mittlere Wiederfindung* von ADMA (Spike) für alle Konzentrationen der Plasmaprobe betrug 103,8% (n=10). Hierzu wurden unterschiedliche Konzentrationen an ADMA den Plasmaproben zugesetzt und die Wiederfindung ermittelt aus den analytisch zu erwartenden und den gemessenen Werten. Die *mittlere Linearität* bei einer Verdünnung der Probe betrug 96% (n=10).

Die Kreuzreaktivität des Antikörpers zu L-Arginin (Konzentation graphisch ermittelt für eine 50%ige Verdrängung am Antikörper) betrug 1:10500 (ADMA / L-Arg) und damit weniger als 0,02%.

Die Kreuzreaktivität zu SDMA (Konzentration bei 75%iger Verdrängung am Antikörper) betrug 1:175 (ADMA/SDMA) und somit weniger als 0,6%.

Insgesamt zeigt Versuch 3, dass damit ein Immunoassay bereitsteht, der an die Erfordernisse von Standard-ELISA-Automaten adaptiert werden kann.

### Beispiel 4 - Kompetitiver Enzymimmunoassay für die Bestimmung von Glycin in humanem EDTA-Plasma

Die Bestimmung erfolgte analog Beispiel 1. Im Einzelnen wurde 50 µL Probe (EDTA-Plasma, Standard beziehungsweise Kontrolle) in 150 µL Phosphatpuffer (pH 8,0) pipettiert und 50 µL 50 mM Boc-Ahx-3,5-Diiodotyrosin-NHS (Formel III), gelöst in DMF zugesetzt, so dass im Reaktionsansatz ein etwa 5facher Überschuss an Derivatisierungsreagenz vorlag. Die Derivatisierung wurde nach 30 Minuten bei Raumtemperatur (18 bis 26°C) durch Zusatz von 250 µl 1%iger BSA gestoppt. Es wurde 50 µL derivatisierte Probe (1:10) in die immunologische Bestimmung eingesetzt. Hierzu wurde derivatisierte Probe mit polyklonalen Antikörpern gegen 3,5-Diiodotyrosin-Glycin in einer mit Biotin-Ahx-Ahx-3,5-Tyr-Glycin (Tracer) beschichteten ELISA-Platte zwei Stunden bei Raumtemperatur (18 bis 26°C) inkubiert. Im Stand der Technik mit acetyliertem Glycin als Zielantigen benötigte die Kompetitionsreaktion bis zur Einstellung des Gleichgewichts über Nacht (15 bis 20 Std) bei 2 bis 8°C. Während der Inkubation konkurriert das Zielantigen in der Probe mit dem an die Platte gebundenen Tracer um die Bindung am polyklonalen Anti-3,5-Diiodotyrosin-Glycin-Antikörper. Hierbei verdrängte das derivatisierte Zielantigen in der Probe den Antikörper aus der Bindung am Tracer. Daher war die Konzentration des an den Tracer gebundenen Antikörpers umgekehrt proportional zur Konzentration des Zielantigens in der Probe. Im zweiten Schritt wurde ein HRP-markierter Sekundärantikörper (1:200 in Konjugatstabilisierungspuffer) zugegeben und 30 Minuten bei Raumtemperatur inkubiert. Nach mehrfachem Waschen zur Entfernung ungebundener Komponenten wurde Peroxidasesubstrat Tetramethylbenzidin (TMB) zugegeben. Die Enzymreaktion wurde wie in Beispiel 1 durch Zugabe von Säure abgestoppt. Dadurch erfolgte ein Farbumschlag von Blau nach Gelb. Die chromogene Verbindung wurde photometrisch bei 450 nm im Mikrotiterplatten-Photometer gegen die Referenzwellenläge (620 nm) gemessen.

Die Intensität ist umgekehrt proportional zur Konzentration des gemessenen Analyten. Mit steigender Glycin-Konzentration in der Probe reduzierte sich die Konzentration der an den Tracer gebundenen Antikörper und das Signal nahm ab. Parallel dazu wurde eine Standardkurve - optische Dichte (Absorption bei 450 nm) versus Standardkonzentration - erstellt, so dass sich die Konzentrationen der Proben durch Vergleich ermitteln ließen. Die Affinität des Antikörpers gegen das 3,5-Tyr-Glycin-Zielantigen war ungleich höher als gegen acyliertes Glycin-Zielantigen, so dass die Bindungsanalyse bei Raumtemperatur und innerhalb zwei Stunden erfolgen konnten.

EDTA-Plasma-Mittelwert: ca 250 µMol/L; Nachweisgrenze: ca. 12-15 µMol/L; Kreuzreaktion mit L-Alanin < 1 %; β-Alanin < 0,2 %.

### Beispiel 5- Automatisierbarer Enzymimmunoassay für die Bestimmung von Histamin in Plasma

Die Bestimmung erfolgte analog Beispiel 2. Es wurde 25 µL Probe (EDTA-Plasma und Kontrolle) in 150 µL Phosphatpuffer (pH 8,0) pipettiert, 25 µL 50 µl 50mM Boc-Ahx-Trp-β-Ala-NHS in DMF zugegeben, gemischt und bei Raumtemperatur (18 bis 26°C) inkubiert. Nach 30 Minuten wurde die Derivatisierung durch Zusatz von 250 µL 1%iger BSA-Lösung gestoppt.

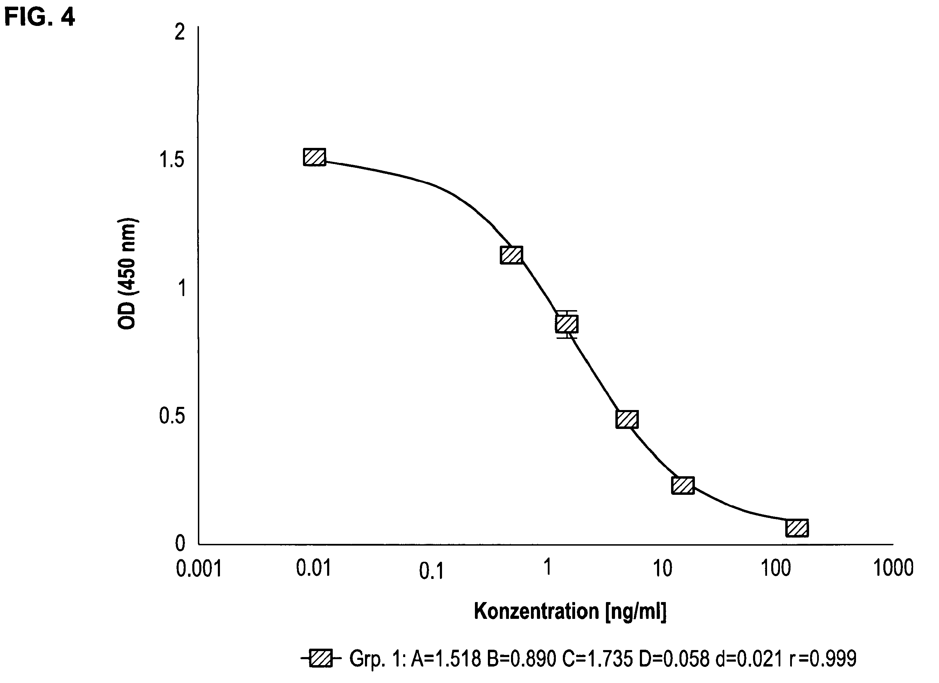

50 µL derivatisierte Probe (1:10), 50 µL Biotin-Ahx-Ahx-Trp-β-Ala-Histamin (Tracer) und 100 µL polyklonaler Kaninchen-Anti-Trp-β-Ala-Histamin-Antikörper (1:10000 verdünnt, gewonnen gegen KLH-DSS-Trp-β-Ala-Histamin)) wurden nur eine einzige (1) Stunde in Assaypuffer bei Raumtemperatur (18 - 26°C) in einer mit Streptavidin beschichteten Mikrotiterplatte inkubiert. Während der Inkubation konkurriert das Histamin-Zielantigen in der derivatisierten Probe mit dem zugesetzten Biotin-Ahx-Ahx-Trp-β-Ala-Histamin-Tracer um die Bindung am polyklonalen Kaninchen-Anti-Trp-β-Ala-Histamin-Antikörper. Hierbei verdrängt das derivatisierte Zielantigen in der Probe den Tracer aus der Bindung am Kaninchen-Antikörper. Daher ist die Konzentration des an der Platte über den Tracer gebundenen Antikörpers umgekehrt proportional zur Konzentration des Histamin-Zielantigens in der Probe. Die feste Phase wurde drei Mal mit Waschpuffer gewaschen und die Menge des an der festen Phase gebundenen Antikörpers bestimmt durch Inkubation (0,5 Std) mit 100 µL POX-konjugiertem Anti-Kaninchen-IgG-Sekundärantikörper (1:50000 in Waschpuffer, entsprechend ca. 100 ng), mehrfachem Abwaschen ungebundener Komponenten und Entwickeln mit 100 µl TMB-Substrat-Lösung (NOVUM Diagnostika GmbH, DE) bei Raumtemperatur (18-26°C) im Dunkeln. Nach 15 Minuten wurde die Farbentwicklung durch Zugabe von 50 µL 2 M H₂SO₄ gestoppt. Die Messung der optischen Dichte erfolgte im Anschluss, wobei die Extinktion im Mikrotiterplatten-Photometer bei 450 nm gegen die Referenzwellenlänge 620 nm gemessen wurde. Die Farbintensität ist umgekehrt proportional zur Konzentration des gemessenen Analyten. Figur 4 zeigt eine übliche Standardkurve für die Bestimmung von Histamin in Serum. Der Normbereich für Histamin in EDTA-Plasma liegt zwischen 0,2 bis 1 ng/mL. Die genannten Reaktionszeiten erlauben somit die Bestimmung von Histamin in einem Laborautomaten.

## Patentansprüche

1. Verfahren zur immunologischen Bestimmung der Konzentration eines niedermolekularen Analyten in einer flüssigen biologischen Probe, wobei der niedermolekulare Analyt eine nicht-peptidische Aminverbindung mit einer Molmasse von weniger als 500 Dalton ist oder eine peptidische Verbindung mit einer reaktiven Aminogruppe und einer Molmasse von weniger als 1500 Dalton, umfassend die Verfahrensschritte:
Zusetzen zu einer Probe mit dem niedermolekularen Analyten im stöchiometrischen Überschuss eines Derivatisierungsmittels, das eine chemisch reaktive Gruppe (RG) enthält, die sich quantitativ mit der Aminogruppe (A) des niedermolekularen Analyten umsetzt, wobei das Derivatisierungsmittel in einem Abstand von weniger als zwölf Kohlenstoffbindungen zur chemisch reaktiven Gruppe (RG) eine Gruppe (GE) mit π-Elektronen oder π-Liganden-Orbitalen aufweist und ausgewählt ist aus Verbindungen der allgemeinen Formeln (la) oder (IIa): 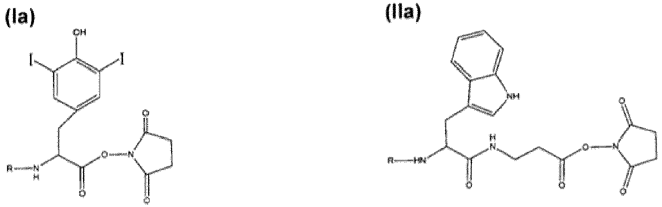 wobei R ein peptidischer Rest ist oder ein Kohlenwasserstoff mit bis zu 40 Kohlenstoffatomen, der Hydroxyl- oder Ketogruppen enthalten kann sowie wahlfrei N, P, O, oder S;
Umsetzen des Analyten in der Probe mit dem Derivatisierungsmittel, optional gefolgt von einem geeigneten Stoppen der Umsetzung mit dem Derivatisierungsmittel, und Erhalt von derivatisiertem Analyt in der Probe;
Bereitstellen von Antikörpern oder Antikörperfragmenten (AK), hergestellt gegen und spezifisch für den derivatisierten Analyt, wobei die Kontaktstelle zwischen den hypervariablen Domänen der variablen Ketten des Antikörpers und dem derivatisierten Analyt die Gruppe (GE) mit den *π*-Elektronen oder *π*-Liganden-Orbitalen sowie die Amingruppe (A) nach ihrer Umsetzung mit der reaktiven Gruppe (RG) des Derivatisierungsmittels umfasst;
Zusammenbringen der Probe mit dem derivatisierten Analyt mit den spezifischen Antikörpern oder Antikörperfragmenten (AK) und Bestimmen der Menge an derivatisiertem Analyt in der Probe durch Bindungsanalyse, und
Bestimmen der Konzentration des niedermolekularen Analyten in der Probe durch Vergleich mit einer Standardreihe,
wobei der Analyt ein biogenes Amin ist und ausgewählt aus der Gruppe mit asymmetrisches Dimethylarginin (ADMA), symmetrisches Dimethylarginin (SDMA), gamma-Aminobuttersäure (GABA), L-Glycin, L-Tryptophan, Amine des Kynureninwegs, L-Arginin, Homocystein, Citrullin, D-Serin, Cystein, Methionin, Carnitin, Phosphoserin, Sulfocystein, Argininobernsteinsäure, Hydroxyprolin, Aspartat, Phosphoethanolamin, Sarcosin (N-Methylglycin), Carnosin (β-Alanyl-L-histidin), Anserine (β-Alanyl-N-methyl-L-histidin), 1,3-Methylhistidin, alpha-Aminoadipinsäure, Methoxytryptamin, beta-Alanin, Prolin, Ethanolamin, beta-Aminoisobuttersäure, L-allo-Cystathionin-A, L-Cystathionin, Cystin, allo-Isoleucin, DL-Hydroxylysin, DL-allo-Hydroxylysin, und Homocystin; Serotonin, Histamin; Glutamin; Katecholamine, Dopamin, Metanephrine, Normetanephrine; cholinerge Amine, Adrenalin, Noradrenalin; Oligopeptide und kleinen Peptidhormonen mit bis zu 12 Aminosäuren; Nonapeptide, Vasopressin Oxytocin; Dekapeptide, Angiotensin-I; Oktapeptide, Angiotensin-II; T3 (Triiodothyronin), und T4 (Thyroxin).

2. Verfahren nach Anspruch 1, wobei der Analyt ausgewählt ist aus ADMA, SDMA, GABA und Glycin.

3. Verfahren nach Anspruch 1 oder 2, umfassend das Zusetzen im stöchiometrischen Überschuss des Derivatisierungsmittels zu einer Blut-, Serum- oder Plasmaprobe eines Patienten.

4. Verfahren nach einem der Ansprüche 1 bis 3, wobei das Derivatisierungsmittel ausgewählt ist aus Verbindungen der nachstehenden Formel (I) oder (II):

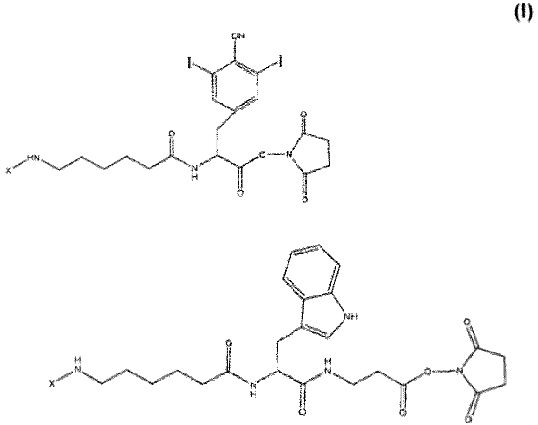

5. Derivatisierungsmittel gemäß nachstehenden Formeln (III) und (IV): 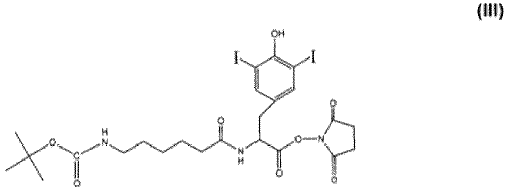 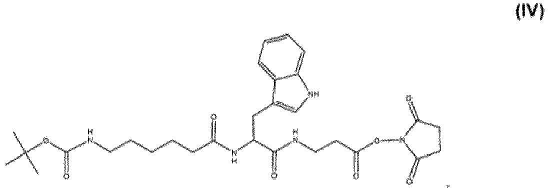 worin ist: X gleich tert-Butyloxycarbonyl (Boc), 9-Fluorenylmethoxycarbonyl (Fmoc), Benzyloxycarbonyl (Cbz), Acetyl, Acyl oder eine Aminschutzgruppe.

6. Derivatisierungsreagenz, enthaltend ein Derivatisierungsmittel, wie in einem der Ansprüche 1 bis 5 definiert, gelöst in einem protischen Lösungsmittel.

7. Reagenziensatz, umfassend ein Derivatisierungsreagenz gemäß Anspruch 6 oder ein Derivatisierungsmittel wie in einem der Ansprüche 1 bis 5 definiert,
einen Kompetitor, erhalten durch Umsetzen des jeweiligen Analyten wie in Anspruch 1 definiert mit dem Derivatisierungsmittel des Derivatisierungsreagenz und gekoppelt an eine Nachweisgruppe oder wahlfrei an eine feste Phase; und
einen Antikörper, der spezifisch, hochaffin ein Antigen bindet, das erhalten wird bei der Umsetzung des Analyten mit dem Derivatisierungsmittel.

8. Kompetitor-Verbindung, erhältlich durch Umsetzen einer Aminverbindung gemäß Anspruch 1 oder 2 mit einem Derivatisierungsmittel gemäß Anspruch 4 oder 5.

9. Antikörper, der an eine antigene Determinante bindet, die erhalten werden kann durch Umsetzen einer Aminverbindung gemäß Anspruch 1 oder 2 mit einem Derivatisierungsmittel gemäß Anspruch 4 oder 5.

10. Antikörper gemäß Anspruch 9 und immobilisiert an eine feste Phase.

## Claims

1. A method for immunological determination of the concentration of a low-molecular-weight analyte in a liquid biological sample, the analyte of lower molecular weight being a non-peptidic amine compound with a molecule weight of less than 500 Dalton or a peptidic compound with a reactive amino group and of a molecular weight of less than 1500 Dalton, comprising the following method steps:
adding to a sample with said low-molecular-weight analyte a stoichiometric excess of derivatizing agent which has a chemically reactive group (RG) quantitatively reacting with an amino group (A) of said low-molecular-weight analyte and which derivatizing agent has within a distance of less than 12 carbon bonds to said chemically reactive group (RG) a group (GE) that has π electrons or π ligand orbitals and selected from compounds of the general formulae (Ia) or (IIa): 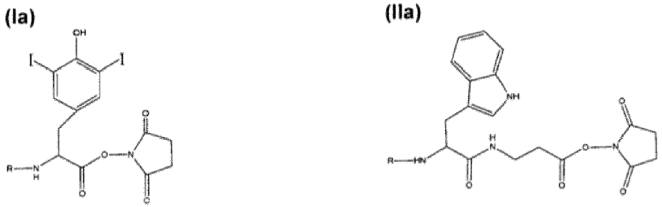 wherein R is a peptidic residue or a hydrocarbon having up to 40 carbon atoms which may comprise hydroxy or keto groups and optionally N, P, O, or S;
reacting said analyte in said sample with said derivatizing agent, optionally followed by a suitable stop of the reaction with said derivatizing reagent, and obtaining a derivatized analyte in said sample;
providing antibodies or antibody fragments (AK), made against and specific for said derivatized analyte, and wherein the contact region between the hypervariable domains of the variable chains of said antibody and the derivatized analyte contains a group (GE) with π electrons or π ligand orbitals as well as the amino group (A) after its reaction with a reactive group (RG) of said derivatizing agent;
contacting said sample with said derivatized analyte with said specific antibodies or antibody fragments (AK) and determining the amount of derivatized analyte in said sample by binding analysis, and
determining the concentration of the low-molecular-weight analyte in said sample by comparison with a series of standards,
wherein the analyte is a biogenic amine and selected from a group comprising asymmetric dimethyl arginine (ADMA), symmetric dimethyl arginine (SDMA), gamma-amino butyric acid (GABA), L-glycine, L-tryptophan, amines of the kynurenine pathway, L-arginine, homocysteine, citrulline, D-serine, cysteine, methionine, carnitine, phosphoserine, sulfocysteine, argininosuccinate, hydroxyproline, aspartate, phosphor ethanolamine, sarcosine (*N*-methylglycine), carnosine (ß-alanyl-L-histidine), anserine (ß-alanyl-N-methyl-L-histidine), 1,3-methylhistidine, alpha-amino adipic acid, methoxytryptamine, beta-alanine, proline, ethanolamine, beta-aminoisobutyric acid, L-allocystathionine-A, L-cystathionine, cystine, alloisoleucine, DL-hydroxylysine, DL-allohydroxylysine, und monocystine; serotonin, histamine; glutamine; catecholamines, dopamine, metanephrine, nor-metanephrine; cholinergic amines, adrenaline, noradrenaline; oligopeptides and small peptide hormones having up to 12 amino acids, nonapeptides, vasopressin, oxytocin; decapeptides, angiotensin-I; octapeptides, angiotensin-II; T3 (triiodothyronine), und T4 (thyroxine).

2. The method of claim 1 wherein the analyte is selected from ADMA, SDMA, GABA, and glycine.

3. The method of claim 1 or claim 2, comprising the addition of a stoichiometric excess of derivatizing reagent to a sample of blood, serum, or plasma from the patient.

4. A method in accordance with any claim 1 to 3, wherein the derivatizing agent is selected from compounds of following formulas (I) or (II): 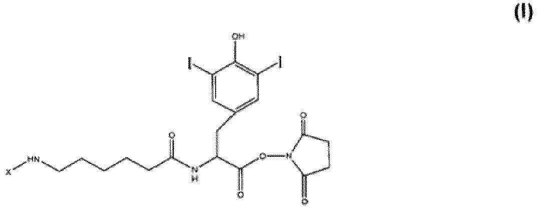 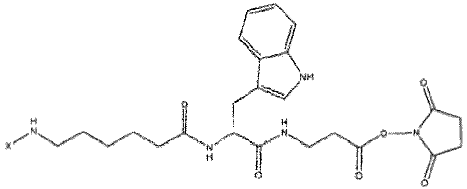 wherein X is tert-butyloxycarbonyl (Boc), 9-fluorenylmethyloxycarbonyl (Fmoc), carbobenzyloxy (Cbz), acetyl, acyl or an amine protecting group.

5. A derivatizing agent of one of the following formulae (III) and (IV):

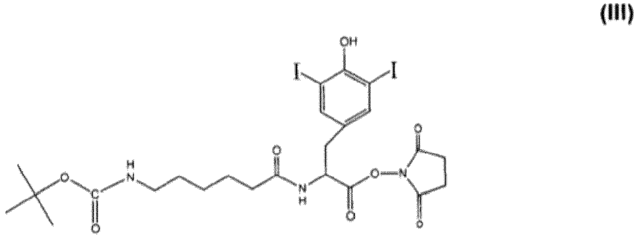

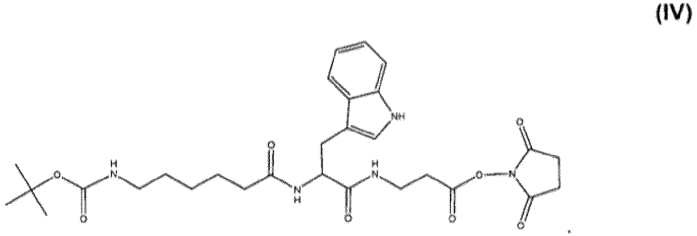

6. A derivatizing reagent comprising a derivatizing agent as defined in any claim 1 to 5 and dissolved in a protic solvent.

7. A set of reagents, comprising a derivatizing reagent in accordance with claim 6, or a derivatizing agent as defined in any claim 1 to 5,
a competitor, obtained by reacting of said respective analyte as defined in claim 1 with said derivatizing agent of said derivatizing reagent and coupled to a detection group or, facultatively, to a stationary phase; and
an antibody which specifically binds an antigen with high affinity which is obtained by the reaction of said analyte with said derivatizing agent.

8. A competitor compound, obtained by reacting an amine compound in accordance with claim 1 or claim 2 with a derivatizing agent as defined in claim 4 or claim 5.

9. An antibody which binds to an antigenic determinant, obtainable by reacting an amine compound in accordance with claim 1 or claim 2 with a derivatizing agent as defined in claim 4 or claim 5.

10. An antibody as defined by claim 9 and immobilized to a stationary phase.

## Revendications

1. Méthode de détermination immunologique de la concentration d'un analyte à faible poids moléculaire dans un échantillon biologique liquide, l'analyte à faible poids moléculaire étant une liaison amine non peptidique d'une masse molaire de moins de 500 daltons ou une liaison peptidique avec un groupe amine réactif et une masse molaire de moins de 1 500 daltons comprenant les étapes de processus :
ajouter à un échantillon avec des analytes à faible poids moléculaire dans un excédent stoechiométrique d'un agent de dérivation qui contient un groupe réactif chimique (RG) et qui s'applique sous forme quantitative au groupe amine (A) de l'analyte à faible poids moléculaire, l'agent de dérivation présentant, à une distance de moins de douze liaisons carbonées avec le groupe réactif chimique (RG), un groupe (GE) avec π-électrons ou π-ligand-orbitaux et qui est choisi à partir d'une liaison des formules générales (la) ou (IIa) : 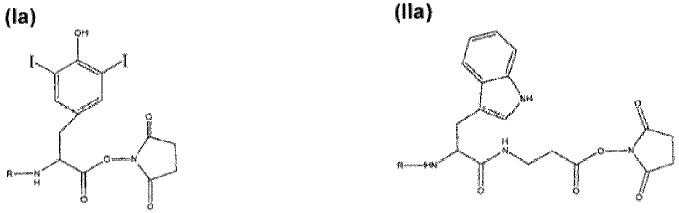 où R est un reste peptidique ou un hydrocarbure contenant jusqu'à 40 atomes de carbone, des groupes hydroxyles ou des groupes cétones ainsi que, à choix, N, P, O ou S;
réaction de l'analyte dans l'échantillon avec un agent de dérivation, suivi en option par un arrêt approprié de la réaction avec l'agent de dérivation, et la réception de l'analyte formé dans l'échantillon ;
mettre en place d'anticorps ou de fragments d'anticorps (AK), fabriqués contre et spécifiquement pour l'analyte formé où a la zone de contact entre les domaines hypervariables des chaînes variables de l'anticorps et d'analyte formé comprend un groupe (GE) avec π-électrons ou π-ligand-orbitaux ainsi que le groupe amine (A) après sa réaction avec le groupe réactif (RG) de l'agent de dérivation ;
mélanger de l'échantillon contenant d'analyte formé avec des anticorps spécifiques ou des fragments d'anticorps (AK) et détermination de la quantité d'analyte formé dans l'échantillon par analyse de la liaison et
détermination de la concentration de l'analyte à faible poids moléculaire dans l'échantillon par comparaison avec une ligne standard,
où l'analyte est une amine biogénique et où il est choisi à partir du groupe avec de l'arginine diméthyle asymétrique (ADMA), arginine diméthyle symétrique (SDMA), acides gamma-aminobutyriques (GABA), L-glycine, L-tryptophane, amines de la voie kynurénine, L-arginine, homocystéine, citrulline, D-sérine, cystéine, méthionine, carnitine, phosphosérine, sulfocystéine, acide argininosuccinique, hydroxyproline, aspartate, phosphoéthanolamine, sarcosine (N-méthylglycine), carnisine (β-alanyle-L-histidine), anserine (β-aianyie-N-méthyie-histidine), 1, 3-méthylhistidine, alpha-acide aminoadipique, méthoxytryptamine, beta-alanine, proline, ethanolamine, bêta-acide aminoadipique, L-allo-cystathionine-A, L-cystathionine, cysteine, allo-isoleucine, DL-hydroxylysine, DL-allo-hydroxylysine et homocystine ; sérotonine, histamine ; glutamine ; catecholamine, dopamine, métanéphrine, normetanéphrine ; amines cholinergène, adrenaline, noradrenaline ; oligopeptides et bas d'hormones peptidique jusque et y compris 12 aminoacides ; nonapeptides, vasopresine, oxytocine : décapeptides, angiotensine-I ; oktapeptides, angiotensine-II ; T3 (triiodothyronine) et T4 (thyroxine).

2. Procédure selon revendication 1 où l'analyte choisi est choisi d'ADMA, SDMA, GABA et glycine.

3. Procédure selon revendication 1 ou 2, comprenant une ajoute en excédent stoechiométrique d'agent de dérivation à un échantillon de sang, de sérum ou de plasma d'un patient.

4. Procédure selon revendication 1 à 3 où l'agent de dérivation est choisi de liaisons des formules (I) ou (II) suivantes : 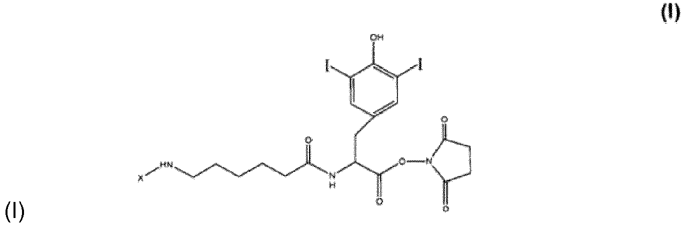 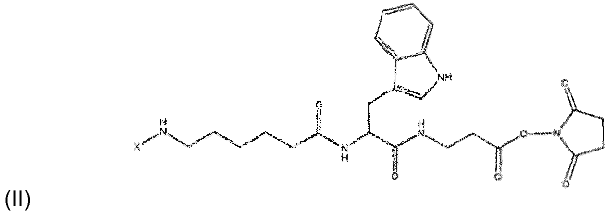 où : X équivaut à du tert-butyloxycarbonyle (Boc), 9-fluorènylméthoxycarbonyle (Fmoc), benzyloxycarbonyle (Cbz), acétyle, acyle ou un groupe amino-protecteur.

5. Agent de dérivation selon les formules (III) et (IV) suivantes :

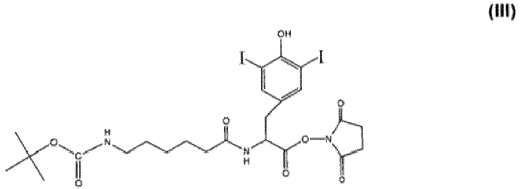

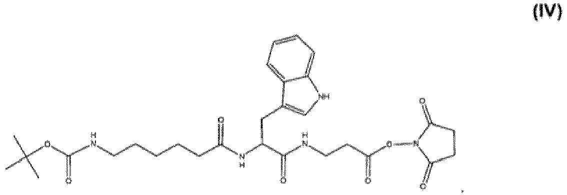

6. Réactif de dérivation contenant un agent de dérivation tel que défini aux revendications 1 à 5, dissous dans un solvant protique.

7. Taux de réactif comprenant un réactif de dérivation conformément à la revendication 6 ou un agent de dérivation tel que défini aux revendications 1 à 5,
un compétiteur obtenu par l'application de l'analyte correspondant tel que défini à la revendication 1 avec un agent de dérivation du réactif de dérivation et couplé à un groupe témoin ou au choix à une phase fixe ; ainsi que
un anticorps qui lie sous forme spécifique un antigène à haute affinité qui sera obtenu lors de la réaction de l'analyte avec l'agent de dérivation.

8. La liaison du compétiteur disponible via la réaction d'une liaison amine conformément aux revendications 1 ou 2 avec un agent de dérivation conformément aux revendications 4 ou 5.

9. L'anticorps qui fait la liaison avec un déterminant antigénique qui peut être obtenu par la réaction d'une liaison amine conformément aux revendications 1 ou 2 avec un autre agent de dérivation conformément aux revendications 4 ou 5.

10. L'anticorps conformément à la revendication 9 immobilisé lors d'une phase solide.
